# EUROPÄISCHE PATENTSCHRIFT

(11) **EP 1 038 014 B1**
(45) Veröffentlichungstag und Bekanntmachung des Hinweises auf die Patenterteilung: **24.08.2005**
(21) Anmeldenummer: 98966266.3
(22) Anmeldetag: 09.12.1998
(51) Int. Cl.: C12N 15/82, C12N 9/10, C07K 16/40, C12N 1/00, A01H 5/00

(54) **PFLANZLICHE (GntI)-SEQUENZEN UND VERWENDUNG DERSELBEN ZUR GEWINNUNG VON PFLANZEN MIT VERMINDERTER ODER FEHLENDER N-ACETYLGLUCOSAMINYLTRANSFERASE I (GnTI)-AKTIVITÄT**
VEGETABLE (GntI) SEQUENCES AND THE USE THEREOF TO OBTAIN PLANTS WITH A REDUCED OR LACK OF N-ACETYLGLUCOSAMINYLTRANSFERASE I (GnTI) ACTIVITY
SEQUENCES DE (GntI) VEGETALES ET UTILISATION DE CELLES-CI POUR L'OBTENTION DE PLANTES NE PRESENTANT PAS UNE ACTIVITE DE N-ACETYLGLUCOSYLAMINYLTRANSFERASE I GnTI OU BIEN SEULEMENT UNE TELLE ACTIVITE REDUITE

(30) Priorität: 09.12.1997 DE 19754622
(43) Veröffentlichungstag der Anmeldung: 27.09.2000
(73) Patentinhaber: von Schaewen, Antje, 49076 Osnabrück (DE)
(72) Erfinder: von Schaewen, Antje, 49076 Osnabrück (DE)
(74) Vertreter: Thurgood, Alexander John
(86) Internationale Anmeldenummer: PCT/EP1998/008001
(87) Internationale Veröffentlichungsnummer: WO 1999/029879

(56) Entgegenhaltungen:
- WO-A-92/09694
- GOMEZ L AND CHRISPEELS M J: "Complementation of an Arabidopsis thaliana mutant that lacks complex asparagine-linked glycans with the human cDNA encoding N-acetylglucosaminyltransferase I" PROCEEDINGS OF THE NATIONAL ACADEMY OF SCIENCES OF THE USA, Bd. 91, Januar 1994, Seiten 1829-1833, XP002100921
- SCHAEWEN VON A ET AL: "ISOLATION OF A MUTANT ARABIDOPSIS PLANT THAT LACKS N-ACETYL GLUCOSAMINYL TRANSFERASE I AND IS UNABLE TO SYNTHESIZE GOLGI- MODIFIED COMPLEX N-LINKED GLYCANS" PLANT PHYSIOLOGY, Bd. 102, Nr. 4, August 1993, Seiten 1109-1118, XP000645269 in der Anmeldung erwähnt

### SEQUENZPROTOKOLL

(1) ALLGEMEINE ANGABEN:
   (i) ANMELDER:
      (A) NAME: von Schaewen, Antje, Dr. rer. nat.
      (B) STRASSE: Natruperstrasse 169a
      (C) ORT: Osnabrück
      (E) LAND: Deutschland
      (F) POSTLEITZAHL: 49076
      (G) TELEFON: 0541-684029
   (ii) BEZEICHNUNG DER ERFINDUNG: Herstellung von Glykoproteinen in Pflanzen mit verminderter oder fehlender N-Acetylglucosaminyltransferase I (GnTI)-Aktivitaet unter Verwendung pflanzlicher gntl-Sequenzen
   (iii) ANZAHL DER SEQUENZEN: 6
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 1669 Basenpaare
      (B) ART: Nucleotid
      (C) STRANGFORM: Doppelstrang
      (D) TOPOLOGIE: linear
   (ii) ART DES MOLEKÜLS: cDNA zu mRNA
   (iii) HYPOTHETISCH: NEIN
   (iv) ANTISENSE: NEIN
   (vi) URSPRÜNLICHE HERKUNFT:
      (A) ORGANISMUS: Solanum tuberosum
      (B) STAMM: Desiree
      (D) ENTWICKLUNGSSTADIUM: "sink" Organ
      (F) GEWEBETYP: Mesophyll
      (G) ZELLTYP: Blattzellen
   (vii) UNMITTELBARE HERKUNFT:
      (A) BIBLIOTHEK: Lambda ZAP II (EcoRI)
      (B) CLON(E) : gntI-A1 (K)
   (ix) MERKMAL:
      (A) NAME/SCHLÜSSEL: misc_feature
      (B) LAGE:659..667
      (D) SONSTIGE ANGABEN: / function= "Asn-Codon ist eine potentielle Glykosylierungsstelle"
         /product= "Konsensus Sequenz fuer N-Glykosylierung"
         /phenotype= "N-Glykane modulieren Proteineigenschaften"
         /standard_name= "N-Glykosylierungsstelle" /label= pot-CHO
         /note= "fehlt in tierischen GnTI-Sequenzen"
   (ix) MERKMAL:
      (A) NAME/SCHLÜSSEL: CDS
      (B) LAGE:53..1393
      (C) ART DER ERMITTLUNG: experimentell
      (D) SONSTIGE ANGABEN:/codon_start= 53
         /function= "initiiert komplexe N-Glykane auf sekret. Glykoproteinen"
         /EC_number= 2.4.1.101
         /product=
         "beta-1,2-N-Acetylglucosaminyltransferase I" /evidence= EXPERIMENTAL
         /gene= "cgl"
         /standard_name= "gntI"
         /label= ORF
         /note= "erste gntI-Sequenz aus Kartoffel (unpubliziert)"
   (ix) MERKMAL:
      (A) NAME/SCHLÜSSEL: 5'UTR
      (B) LAGE:15..52
   (ix) MERKMAL:
      (A) NAME/SCHLÜSSEL: 3'UTR
      (B) LAGE:1394..1655
   (ix) MERKMAL:
      (A) NAME/SCHLÜSSEL: CDS
      (B) LAGE:80..139
      (D) SONSTIGE ANGABEN:/function= "Membrananker (Aminosäuren 10-29)"
         /product= "hydrophober Aminosäurebereich in GnTI"
         /standard_name= "Membrananker eines Typ II Golgi-Proteins"
         /note= "durch Vergleich mit tierischen GnTI-Sequenzen identifiziert"
   (ix) MERKMAL:
      (A) NAME/SCHLÜSSEL: misc_feature
      (B) LAGE:1..14
      (D) SONSTIGE ANGABEN:/function= "zur Herstellung der cDNA-Bank in Lambda ZAP II"
         /product= "EcoRI/NotI-cDNA-Adaptor"
         /number= 1
   (ix) MERKMAL:
      (A) NAME/SCHLÜSSEL: misc_feature
      (B) LAGE:1656..1669
      (D) SONSTIGE ANGABEN:/product= "EcoRI/NotI-Adaptor" /number= 2
   (xi) SEQUENZBESCHREIBUNG: SEQ ID NO: 1:
   GAATTCGCGG CCGCCTGAGA AACCCTCGAA TTCAATTTCG CATTTGGCAG AG ATG           55
                                                             Met
                                                               1
   AGA GGG AAC AAG TTT TGC TTT GAT TTA CGG TAC CTT CTC GTC GTG GCT        103
   Arg Gly Asn Lys Phe Cys Phe Asp Leu Arg Tyr Leu Leu Val Val Ala
               5                   10                  15
   GCT CTC GCC TTC ATC TAC ATA CAG ATG CGG CTT TTC GCG ACA CAG TCA        151
   Ala Leu Ala Phe Ile Tyr Ile Gln Met Arg Leu Phe Ala Thr Gln Ser
           20                  25                  30
   GAA TAT GTA GAC CGC CTT GCT GCT GCA ATT GAA GCA GAA AAT CAT TGT        199
   Glu Tyr Val Asp Arg Leu Ala Ala Ala Ile Glu Ala Glu Asn His Cys
       35                  40                  45
   ACA AGT CAG ACC AGA TTG CTT ATT GAC AAG ATT AGC CAG CAG CAA GGA        247
   Thr Ser Gln Thr Arg Leu Leu Ile Asp Lys Ile Ser Gln Gln Gln Gly
   50                  55                  60                  65
   AGA GTA GTA GCT CTT GAA GAA CAA ATG AAG CAT CAG GAC CAG GAG TGC        295
   Arg Val Val Ala Leu Glu Glu Gln Met Lys His Gln Asp Gln Glu Cys
                   70                  75                  80
   CGG CAA TTA AGG GCT CTT GTT CAG GAT CTT GAA AGT AAG GGC ATA AAA        343
   Arg Gln Leu Arg Ala Leu Val Gln Asp Leu Glu Ser Lys Gly Ile Lys
               85                  90                  95
   AAG TTA ATC GGA GAT GTG CAG ATG CCA GTG GCA GCT GTA GTT GTT ATG        391
   Lys Leu Ile Gly Asp Val Gln Met Pro Val Ala Ala Val Val Val Met
           100                 105                 110
   GCT TGC AGT CGT ACT GAC TAC CTG GAG AGG ACT ATT AAA TCC ATC TTA        439
   Ala Cys Ser Arg Thr Asp Tyr Leu Glu Arg Thr Ile Lys Ser Ile Leu
       115                 120                 125
   AAA TAC CAA ACA TCT GTT GCA TCA AAA TAT CCT CTT TTC ATA TCC CAG        487
   Lys Tyr Gln Thr Ser Val Ala Ser Lys Tyr Pro Leu Phe Ile Ser Gln
   130                 135                 140                 145
   GAT GGA TCA AAT CCT GAT GTA AGA AAG CTT GCT TTG AGC TAT GGT CAG        535
   Asp Gly Ser Asn Pro Asp Val Arg Lys Leu Ala Leu Ser Tyr Gly Gln
                   150                 155                 160
   CTG ACG TAT ATG CAG CAC TTG GAT TAT GAA CCT GTG CAT ACT GAA AGA        583
   Leu Thr Tyr Met Gln His Leu Asp Tyr Glu Pro Val His Thr Glu Arg
               165                 170                 175
   CCA GGG GAA CTG GTT GCA TAC TAC AAG ATT GCA CGT CAT TAC AAG TGG        631
   Pro Gly Glu Leu Val Ala Tyr Tyr Lys Ile Ala Arg His Tyr Lys Trp
           180                 185                 190
   GCA TTG GAT CAG CTG TTT CAC AAG CAT AAT TTT AGC CGT GTT ATC ATA        679
   Ala Leu Asp Gln Leu Phe His Lys His Asn Phe Ser Arg Val Ile Ile
       195                 200                 205
   CTA GAA GAT GAT ATG GAA ATT GCT GCT GAT TTT TTT GAC TAT TTT GAG      727
   Leu Glu Asp Asp Met Glu Ile Ala Ala Asp Phe Phe Asp Tyr Phe Glu
   210                 215                 220                 225
   GCT GGA GCT ACT CTT CTT GAC AGA GAC AAG TCG ATT ATG GCT ATT TCT      775
   Ala Gly Ala Thr Leu Leu Asp Arg Asp Lys Ser Ile Met Ala Ile Ser
                   230                 235                 240
   TCT TGG AAT GAC AAT GGA CAA AGG CAG TTC GTC CAA GAT CCT GAT GCT      823
   Ser Trp Asn Asp Asn Gly Gln Arg Gln Phe Val Gln Asp Pro Asp Ala
               245                 250                 255
   CTT TAC CGC TCA GAC TTT TTT CCT GGT CTT GGA TGG ATG CTT TCA AAA      871
   Leu Tyr Arg Ser Asp Phe Phe Pro Gly Leu Gly Trp Met Leu Ser Lys
           260                 265                 270
   TCA ACT TGG TCC GAA CTA TCT CCA AAG TGG CCA AAG GCT TAC TGG GAT      919
   Ser Thr Trp Ser Glu Leu Ser Pro Lys Trp Pro Lys Ala Tyr Trp Asp
       275                 280                 285
   GAC TGG CTA AGG CTG AAA GAA AAT CAC AGA GGT CGA CAA TTT ATT CGC      967
   Asp Trp Leu Arg Leu Lys Glu Asn His Arg Gly Arg Gln Phe Ile Arg
   290                 295                 300                 305
   CCA GAA GTT TGC AGA ACG TAC AAT TTT GGT GAG CAT GGT TCT AGT TTG     1015
   Pro Glu Val Cys Arg Thr Tyr Asn Phe Gly Glu His Gly Ser Ser Leu
                   310                 315                 320
   GGG CAG TTT TTT AAG CAG TAT CTT GAG CCA ATT AAG CTA AAT GAT GTC     1063
   Gly Gln Phe Phe Lys Gln Tyr Leu Glu Pro Ile Lys Leu Asn Asp Val
               325                 330                 335
   CAG GTT GAT TGG AAG TCA ATG GAC CTA AGT TAC CTT TTG GAG GAC AAC     1111
   Gln Val Asp Trp Lys Ser Met Asp Leu Ser Tyr Leu Leu Glu Asp Asn
           340                 345                 350
   TAT GTG AAA CAC TTT GGC GAC TTG GTT AAA AAG GCT AAG CCC ATC CAC     1159
   Tyr Val Lys His Phe Gly Asp Leu Val Lys Lys Ala Lys Pro Ile His
       355                 360                 365
   GGA GCT GAT GCT GTT TTG AAA GCA TTT AAC ATA GAT GGT GAT GTG CGT     1207
   Gly Ala Asp Ala Val Leu Lys Ala Phe Asn Ile Asp Gly Asp Val Arg
   370                 375                 380                 385
   ATT CAG TAC AGA GAC CAA CTA GAC TTT GAA GAT ATC GCT CGA CAG TTT     1255
   Ile Gln Tyr Arg Asp Gln Leu Asp Phe Glu Asp Ile Ala Arg Gln Phe
                   390                 395                 400
   GGC ATT TTT GAA GAA TGG AAG GAT GGT GTA CCA CGG GCA GCA TAT AAA     1303
   Gly Ile Phe Glu Glu Trp Lys Asp Gly Val Pro Arg Ala Ala Tyr Lys
               405                 410                 415
   GGG ATA GTA GTT TTC CGG TTT CAA ACA TCT AGA CGT GTG TTC CTT GTT     1351
   Gly Ile Val Val Phe Arg Phe Gln Thr Ser Arg Arg Val Phe Leu Val
           420                 425                 430
   TCC CCT GAT TCT CTT CGA CAA CTT GGA GTT GAA GAT ACT TAG             1393
   Ser Pro Asp Ser Leu Arg Gln Leu Gly Val Glu Asp Thr  *
       435                 440                 445
   CGAAGATATG ATTGGAGCCT GAGCAACAAT TTAGACTTAT TTGGTAGGAT ACATTTGAAA   1453
   GAGCTGACAC GAAAAGTATG ACTACCAGTA GCTACATGCA ACATTTTAAT GTTAATGGAA   1513
   GGAACCCACT GCTTATTGTT GGAATGGATG AATCATCACC ACATCCTATT ATTCAAGTTT   1573
   ACAAACATAA AGAGGAAATG TTGCCCTATA AAAACAAATT TTTTGTTTCT AAGAAGGAAC    1633
   GTTACGATTA TGAGCAACTT TGGCGGCCGC GAATTC                              1669
(2) ANGABEN ZU SEQ ID NO: 2:
   (i) SEQUENZKENNZEICHEN:
      (A) LÄNGE: 447 Aminosäuren
      (B) ART: Aminosäure
      (D) TOPOLOGIE: linear
   (ii) ART DES MOLEKULS: Protein
   (xi) SEQUENZBESCHREIBUNG: SEQ ID NO: 2:
   Met Arg Gly Asn Lys Phe Cys Phe Asp Leu Arg Tyr Leu Leu Val Val
    1               5                  10                  15
   Ala Ala Leu Ala Phe Ile Tyr Ile Gln Met Arg Leu Phe Ala Thr Gln
               20                  25                  30
   Ser Glu Tyr Val Asp Arg Leu Ala Ala Ala Ile Glu Ala Glu Asn His
           35                  40                  45
   Cys Thr Ser Gln Thr Arg Leu Leu Ile Asp Lys Ile Ser Gln Gln Gln
       50                  55                  60
   Gly Arg Val Val Ala Leu Glu Glu Gln Met Lys His Gln Asp Gln Glu
   65                  70                  75                  80
   Cys Arg Gln Leu Arg Ala Leu Val Gln Asp Leu Glu Ser Lys Gly Ile
                   85                  90                  95
   Lys Lys Leu Ile Gly Asp Val Gln Met Pro Val Ala Ala Val Val Val
               100                 105                 110
   Met Ala Cys Ser Arg Thr Asp Tyr Leu Glu Arg Thr Ile Lys Ser Ile
           115                 120                 125
   Leu Lys Tyr Gln Thr Ser Val Ala Ser Lys Tyr Pro Leu Phe Ile Ser
       130                 135                 140
   Gln Asp Gly Ser Asn Pro Asp Val Arg Lys Leu Ala Leu Ser Tyr Gly
   145                 150                 155                 160
   Gln Leu Thr Tyr Met Gln His Leu Asp Tyr Glu Pro Val His Thr Glu
                   165                 170                 175
   Arg Pro Gly Glu Leu Val Ala Tyr Tyr Lys Ile Ala Arg His Tyr Lys
               180                 185                 190
   Trp Ala Leu Asp Gln Leu Phe His Lys His Asn Phe Ser Arg Val Ile
           195                 200                 205
   Ile Leu Glu Asp Asp Met Glu Ile Ala Ala Asp Phe Phe Asp Tyr Phe
       210                 215                 220
   Glu Ala Gly Ala Thr Leu Leu Asp Arg Asp Lys Ser Ile Met Ala Ile
   225                 230                 235                 240
   Ser Ser Trp Asn Asp Asn Gly Gln Arg Gln Phe Val Gln Asp Pro Asp
                   245                 250                 255
   Ala Leu Tyr Arg Ser Asp Phe Phe Pro Gly Leu Gly Trp Met Leu Ser
               260                 265                 270
   Lys Ser Thr Trp Ser Glu Leu Ser Pro Lys Trp Pro Lys Ala Tyr Trp
           275                 280                 285
   Asp Asp Trp Leu Arg Leu Lys Glu Asn His Arg Gly Arg Gln Phe Ile
       290                 295                 300
   Arg Pro Glu Val Cys Arg Thr Tyr Asn Phe Gly Glu His Gly Ser Ser
   305                 310                 315                 320
   Leu Gly Gln Phe Phe Lys Gln Tyr Leu Glu Pro Ile Lys Leu Asn Asp
                   325                 330                 335
   Val Gln Val Asp Trp Lys Ser Met Asp Leu Ser Tyr Leu Leu Glu Asp
               340                 345                 350
   Asn Tyr Val Lys His Phe Gly Asp Leu Val Lys Lys Ala Lys Pro Ile
           355                 360                 365
   His Gly Ala Asp Ala Val Leu Lys Ala Phe Asn Ile Asp Gly Asp Val
       370                 375                 380
   Arg Ile Gln Tyr Arg Asp Gln Leu Asp Phe Glu Asp Ile Ala Arg Gln
   385                 390                 395                 400
   Phe Gly Ile Phe Glu Glu Trp Lys Asp Gly Val Pro Arg Ala Ala Tyr
                   405                 410                 415
   Lys Gly Ile Val Val Phe Arg Phe Gln Thr Ser Arg Arg Val Phe Leu
               420                 425                 430
   Val Ser Pro Asp Ser Leu Arg Gln Leu Gly Val Glu Asp Thr  *
           435                 440                 445
(2) ANGABEN ZU SEQ ID NO: 3:
   (i) SEQUENZKENNZEICHEN:
      (A) LANGE: 1737 Basenpaare
      (B) ART: Nucleotid
      (C) STRANGFORM: Doppelstrang
      (D) TOPOLOGIE: linear
   (ii) ART DES MOLEKÜLS: cDNA zu mRNA
   (iii) HYPOTHETISCH: NEIN
   (iv) ANTISENSE: NEIN
   (vi) URSPRÜNGLICHE HERKUNFT:
      (A) ORGANISMUS: Nicotiana tabacum
      (B) STAMM: Samsun NN
      (D) ENTWICKLUNGSSTADIUM: "sink" Organ
      (F) GEWEBETYP: Mesophyll
      (G) ZELLTYP: Blattzellen
   (vii) UNMITTELBARE HERKUNFT:
      (A) BIBLIOTHEK: Lambda ZAP II (EcoRI)
      (B) CLON(E): gntI-A9(T)
   (ix) MERKMAL:
      (A) NAME/SCHLÜSSEL: misc_feature
      (B) LAGE:733..741
      (D) SONSTIGE ANGABEN:/function= "Asn-Codon ist eine potentielle Glykosylierungsstelle"
         /product= "Konsensus Sequenz für N-Glykosylierung"
         /phenotype= "N-Glykane modulieren Proteineigenschaften"
         /standard_name= "N-Glykosylierungsstelle" /label= pot-CHO
         /note= "fehlt in tierischen GnTI-Sequenzen"
   (ix) MERKMAL:
      (A) NAME/SCHLÜSSEL: CDS
      (B) LAGE:127..1467
      (C) ART DER ERMITTLUNG: experimentell
      (D) SONSTIGE ANGABEN:/codon_start= 127
         /function= "initiiert komplexe N-Glykane auf sekret. Glykoproteinen"
         /EC_number= 2.4.1.101
         /product=
         "beta-1,2-N-Acetylglucosaminyltransferase I" /evidence= EXPERIMENTAL
         /gene= "cgl"
         /standard_name= "gntI"
         /label= ORF
         /note= "erste gntI-Sequenz aus Tabak (unpubliziert)"
   (ix) MERKMAL:
      (A) NAME/SCHLÜSSEL: 5'UTR
      (B) LAGE:15..126
   (ix) MERKMAL:
      (A) NAME/SCHLÜSSEL: 3'UTR
      (B) LAGE:1468..1723
   (ix) MERKMAL:
      (A) NAME/SCHLÜSSEL: CDS
      (B) LAGE:154..213
      (D) SONSTIGE ANGABEN:/function= "Membrananker (Aminosäuren 10-29)"
         /product= "hydrophober Aminosäurebereich in GnTI"
         /standard_name= "Membrananker eines Typ II Golgi-Proteins"
         /note= "durch Vergleich mit tierischen GnTI-Sequenzen identifiziert"
   (ix) MERKMAL:
      (A) NAME/SCHLÜSSEL: misc_feature
      (B) LAGE:1..14
      (D) SONSTIGE ANGABEN:/function= "zur Herstellung der cDNA-Bank in Lambda ZAP II"
         /product= "EcoRI/NotI-cDNA-Adaptor"
         /number= 1
   (ix) MERKMAL:
      (A) NAME/SCHLÜSSEL: misc_feature
      (B) LAGE:1724..1737
      (D) SONSTIGE ANGABEN:/product= "EcoRI/NotI-Adaptor" /number= 2
   (xi) SEQUENZBESCHREIBUNG: SEQ ID NO: 3:
   GAATTCGCGG CCGCCATTGA CTTGATCCTA ACTGAACAGG CAAAGTAAAT CCAGCGATGA      60
   AACACTCATA ACTGAACACT GAGAGACTAT TCGCTTTCTC CTAAAGCCTT CAATCGAATT     120
   CGCACG ATG AGA GGG AAC AAG TTT TGC TGT GAT TTC CGG TAC CTC CTC        168
          Met Arg Gly Asn Lys Phe Cys Cys Asp Phe Arg Tyr Leu Leu
                  450                 455                 460
   ATC TTG GCT GCT GTC GCC TTC ATC TAC ACA CAG ATG CGG CTT TTT GCG       216
   Ile Leu Ala Ala Val Ala Phe Ile Tyr Thr Gln Met Arg Leu Phe Ala
               465                 470                 475
   ACA CAG TCA GAA TAT GCA GAT CGC CTT GCT GCT GCA ATT GAA GCA GAA       264
   Thr Gln Ser Glu Tyr Ala Asp Arg Leu Ala Ala Ala Ile Glu Ala Glu
           480                 485                 490
   AAT CAT TGT ACA AGC CAG ACC AGA TTG CTT ATT GAC CAG ATT AGC CTG       312
   Asn His Cys Thr Ser Gln Thr Arg Leu Leu Ile Asp Gln Ile Ser Leu
       495                 500                 505
   CAG CAA GGA AGA ATA GTT GCT CTT GAA GAA CAA ATG AAG CGT CAG GAC       360
   Gln Gln Gly Arg Ile Val Ala Leu Glu Glu Gln Met Lys Arg Gln Asp
   510                 515                 520                 525
   CAG GAG TGC CGA CAA TTA AGG GCT CTT GTT CAG GAT CTT GAA AGT AAG       408
   Gln Glu Cys Arg Gln Leu Arg Ala Leu Val Gln Asp Leu Glu Ser Lys
                   530                 535                 540
   GGC ATA AAA AAG TTG ATC GGA AAT GTA CAG ATG CCA GTG GCT GCT GTA       456
   Gly Ile Lys Lys Leu Ile Gly Asn Val Gln Met Pro Val Ala Ala Val
               545                 550                 555
   GTT GTT ATG GCT TGC AAT CGG GCT GAT TAC CTG GAA AAG ACT ATT AAA       504
   Val Val Met Ala Cys Asn Arg Ala Asp Tyr Leu Glu Lys Thr Ile Lys
           560                 565                 570
   TCC ATC TTA AAA TAC CAA ATA TCT GTT GCG TCA AAA TAT CCT CTT TTC       552
   Ser Ile Leu Lys Tyr Gln Ile Ser Val Ala Ser Lys Tyr Pro Leu Phe
       575                 580                 585
   ATA TCC CAG GAT GGA TCA CAT CCT GAT GTC AGG AAG CTT GCT TTG AGC       600
   Ile Ser Gln Asp Gly Ser His Pro Asp Val Arg Lys Leu Ala Leu Ser
   590                 595                 600                 605
   TAT GAT CAG CTG ACG TAT ATG CAG CAC TTG GAT TTT GAA CCT GTG CAT       648
   Tyr Asp Gln Leu Thr Tyr Met Gln His Leu Asp Phe Glu Pro Val His
                   610                 615                 620
   ACT GAA AGA CCA GGG GAG CTG ATT GCA TAC TAC AAA ATT GCA CGT CAT       696
   Thr Glu Arg Pro Gly Glu Leu Ile Ala Tyr Tyr Lys Ile Ala Arg His
               625                 630                 635
   TAC AAG TGG GCA TTG GAT CAG CTG TTT TAC AAG CAT AAT TTT AGC CGT      744
   Tyr Lys Trp Ala Leu Asp Gln Leu Phe Tyr Lys His Asn Phe Ser Arg
           640                 645                 650
   GTT ATC ATA CTA GAA GAT GAT ATG GAA ATT GCC CCT GAT TTT TTT GAC      792
   Val Ile Ile Leu Glu Asp Asp Met Glu Ile Ala Pro Asp Phe Phe Asp
       655                 660                 665
   TTT TTT GAG GCT GGA GCT ACT CTT CTT GAC AGA GAC AAG TCG ATT ATG      840
   Phe Phe Glu Ala Gly Ala Thr Leu Leu Asp Arg Asp Lys Ser Ile Met
   670                 675                 680                 685
   GCT ATT TCT TCT TGG AAT GAC AAT GGA CAA ATG CAG TTT GTC CAA GAT      888
   Ala Ile Ser Ser Trp Asn Asp Asn Gly Gln Met Gln Phe Val Gln Asp
                   690                 695                 700
   CCT TAT GCT CTT TAC CGC TCA GAT TTT TTT CCC GGT CTT GGA TGG ATG      936
   Pro Tyr Ala Leu Tyr Arg Ser Asp Phe Phe Pro Gly Leu Gly Trp Met
               705                 710                 715
   CTT TCA AAA TCT ACT TGG GAC GAA TTA TCT CCA AAG TGG CCA AAG GCT      984
   Leu Ser Lys Ser Thr Trp Asp Glu Leu Ser Pro Lys Trp Pro Lys Ala
           720                 725                 730
   TAC TGG GAC GAC TGG CTA AGA CTC AAA GAG AAT CAC AGA GGT CGA CAA     1032
   Tyr Trp Asp Asp Trp Leu Arg Leu Lys Glu Asn His Arg Gly Arg Gln
       735                 740                 745
   TTT ATT CGC CCA GAA GTT TGC AGA ACA TAT AAT TTT GGT GAG CAT GGT     1080
   Phe Ile Arg Pro Glu Val Cys Arg Thr Tyr Asn Phe Gly Glu His Gly
   750                 755                 760                 765
   TCT AGT TTG GGG CAG TTT TTC AAG CAG TAT CTT GAG CCA ATT AAA CTA     1128
   Ser Ser Leu Gly Gln Phe Phe Lys Gln Tyr Leu Glu Pro Ile Lys Leu
                   770                 775                 780
   AAT GAT GTC CAG GTT GAT TGG AAG TCA ATG GAC CTT AGT TAC CTT TTG     1176
   Asn Asp Val Gln Val Asp Trp Lys Ser Met Asp Leu Ser Tyr Leu Leu
               785                 790                 795
   GAG GAC AAT TAC GTG AAA CAC TTT GGT GAC TTG GTT AAA AAG GCT AAG     1224
   Glu Asp Asn Tyr Val Lys His Phe Gly Asp Leu Val Lys Lys Ala Lys
           800                 805                 810
   CCC ATC CAT GGA GCT GAT GCT GTC TTG AAA GCA TTT AAC ATA GAT GGT     1272
   Pro Ile His Gly Ala Asp Ala Val Leu Lys Ala Phe Asn Ile Asp Gly
       815                 820                 825
   GAT GTG CGT ATT CAG TAC AGA GAT CAA CTA GAC TTT GAA AAT ATC GCA     1320
   Asp Val Arg Ile Gln Tyr Arg Asp Gln Leu Asp Phe Glu Asn Ile Ala
   830                 835                 840                 845
   CGG CAA TTT GGC ATT TTT GAA GAA TGG AAG GAT GGT GTA CCA CGT GCA     1368
   Arg Gln Phe Gly Ile Phe Glu Glu Trp Lys Asp Gly Val Pro Arg Ala
                   850                 855                 860
   GCA TAT AAA GGA ATA GTA GTT TTC CGG TAC CAA ACG TCC AGA CGT GTA     1416
   Ala Tyr Lys Gly Ile Val Val Phe Arg Tyr Gln Thr Ser Arg Arg Val
               865                 870                 875
   TTC CTT GTT GGC CAT GAT TCG CTT CAA CAA CTC GGA ATT GAA GAT ACT     1464
   Phe Leu Val Gly His Asp Ser Leu Gln Gln Leu Gly Ile Glu Asp Thr
           880                 885                 890
   TAA CAAAGATATG ATTGCAGGAG CCCGGGCAAA ATTTTTGACT TATTGGGTAG          1517
   GATGCATCGA GCTGACACTA AACCATGATT TTACCAGTTA CATACAACGT TTTAATGTTA    1577
   TACGGAGGAG CTCACTGTTC TAGTGTTGAA GGGATATCGG CTTCTTAGTA TTGGATGAAT    1637
   CATCAACACA ACCTATTATT TTAAGTGTTC AGAACATAAA GAGGAAATGT AGCCCTGTAA    1697
   AGACTATACA TGGGACCATC ATAATCGCGG CCGCGAATTC                          1737
(2) ANGABEN ZU SEQ ID NO: 4:
   (i) SEQUENZKENNZEICHEN:
      (A) LÄNGE; 447 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
(2) ANGABEN ZU SEQ ID NO: 5:
   (i) SEQUENZKENNZEICHEN:
      (A) LÄNGE: 1854 Basenpaare
      (B) ART: Nucleotid
      (C) STRANGFORM: Doppelstrang
      (D) TOPOLOGIE: linear
   (ii) ART DES MOLEKÜLS: cDNA zu mRNA
   (iii) HYPOTHETISCH: NEIN
   (iv) ANTISENSE: NEIN
   (vi) URSPRÜNLICHE HERKUNFT:
      (A) ORGANISMUS: Arabidopsis thaliana.
      (B) STAMM: Columbia
      (D) ENTWICKLUNGSSTADIUM: reife Pflanzen
      (F) GEWEBETYP: alle Gewebe
   (vii) UNMITTELBARE HERKUNFT:
      (A) BIBLIOTHEK: Lambda Uni-ZAP (EcoRI/XhoI) und Lambda ACT (XhoI)
      (B) CLON(E): pBSK-Ara-GntI-full #8
   (ix) MERKMAL:
      (A) NAME/SCHLÜSSEL: misc_feature
      (B) LAGE:1185..1193
      (D) SONSTIGE ANGABEN:/function= "Asn-Codon ist eine potentielle Glykosylierungsstelle"
         /product= "Konsensus Sequenz fuer N-Glykosylierung"
         /phenotype= "N-Glykane modulieren Proteineigenschaften"
         /standard name= "N-Glykosylierungsstelle" /label= pot-CHO
         /note= "fehlt in tierischen GnTI-Sequenzen"
   (ix) MERKMAL:
      (A) NAME/SCHLÜSSEL: CDS
      (B) LAGE:135..1469
      (C) ART DER ERMITTLUNG: experimentell
      (D) SONSTIGE ANGABEN:/codon_start= 135
         /function= "initiiert komplexe N-Glykane auf sekret. Glykoproteinen"
         /EC_number= 2.4.1.101
         /product=
         "beta-1,2-N-Acetylglucosaminyltransferase I" /evidence= EXPERIMENTAL
         /gene= "cgl"
         /standard_name= "gntI"
         /label= ORF
         /note= "erste gntI-Sequenz aus Arabidopsis (unpubliziert)"
   (ix) MERKMAL:
      (A) NAME/SCHLÜSSEL: 5'UTR
      (B) LAGE:19..134
   (ix) MERKMAL:
      (A) NAME/SCHLÜSSEL: 3'UTR
      (B) LAGE:1470..1848
   (ix) MERKMAL:
      (A) NAME/SCHLÜSSEL: CDS
      (B) LAGE:157..215
      (D) SONSTIGE ANGABEN:/function= "Membrananker (Aminosäuren 8-27)"
         /product= "hydrophober Aminosäurebereich in GnTI"
         /standard_name= "Membrananker eines Typ II Golgi-Proteins"
         /note= "durch Vergleich mit tierischen GnTI-Sequenzen identifiziert"
   (ix) MERKMAL:
      (A) NAME/SCHLÜSSEL: misc_feature
      (B) LAGE:1..18
      (D) SONSTIGE ANGABEN:/function= "zur Herstellung einer cDNA-Bibliothek in Lambda ACT"
         /product= "XhoI-cDNA-Adaptor"
         /number= 1
   (ix) MERKMAL:
      (A) NAME/SCHLÜSSEL: misc_feature
      (B) LAGE:1849..1854
      (D) SONSTIGE ANGABEN:/product= "XhoI-cDNA-Adaptor" /number= 2
   (xi) SEQUENZBESCHREIBUNG: SEQ ID NO: 5:
   CTCGAGGCCA CGAAGGCCAC CGTTTTTGTT ATAACGAACG ACACCGTTTC AAACAACTTC      60
   CTTATTAGCT AGCTCCCTCC CGGCGGCAAA CACCAGAAGA TCCACCGCTT TTGATCTGGT     120
   TGTTTGTCGT CGAT ATG GCG AGG ATC TCG TGT GAC TTG AGA TTT CTT CTC       170
                   Met Ala Arg Ile Ser Cys Asp Leu Arg Phe Leu Leu
                    1               5                  10
   ATC CCG GCA GCT TTC ATG TTC ATC TAC ATC CAG ATG AGG CTT TTC CAG       218
   Ile Pro Ala Ala Phe Met Phe Ile Tyr Ile Gln Met Arg Leu Phe Gln
           15                  20                  25
   ACG CAA TCA CAG TAT GCA GAT CGC CTC AGT TCC GCT ATC GAA TCT GAG       266
   Thr Gln Ser Gln Tyr Ala Asp Arg Leu Ser Ser Ala Ile Glu Ser Glu
       30                  35                  40
   AAC CAT TGC ACT AGT CAA ATG CGA GGC CTC ATA GAT GAA GTT AGC ATC       314
   Asn His Cys Thr Ser Gln Met Arg Gly Leu Ile Asp Glu Val Ser Ile
   45                  50                  55                  60
   AAA CAG TCG CGG ATT GTT GCC CTC GAA GAT ATG AAG AAC CGC CAG GAC       362
   Lys Gln Ser Arg Ile Val Ala Leu Glu Asp Met Lys Asn Arg Gln Asp
                   65                  70                  75
   GAA GAA CTT GTG CAG CTT AAG GAT CTA ATC CAG ACG TTT GAA AAA AAA       410
   Glu Glu Leu Val Gln Leu Lys Asp Leu Ile Gln Thr Phe Glu Lys Lys
               80                  85                  90
   GGA ATA GCA AAA CTC ACT CAA GGT GGA CAG ATG CCT GTG GCT GCT GTA       458
   Gly Ile Ala Lys Leu Thr Gln Gly Gly Gln Met Pro Val Ala Ala Val
           95                  100                 105
   GTG GTT ATG GCC TGC AGT CGT GCA GAC TAT CTT GAA AGG ACT GTT AAA       506
   Val Val Met Ala Cys Ser Arg Ala Asp Tyr Leu Glu Arg Thr Val Lys
       110                 115                 120
   TCA GTT TTA ACA TAT CAA ACT CCC GTT GCT TCA AAA TAT CCT CTA TTT       554
   Ser Val Leu Thr Tyr Gln Thr Pro Val Ala Ser Lys Tyr Pro Leu Phe
   125                 130                 135                 140
   ATA TCT CAG GAT GGA TCT GAT CAA GCT GTC AAG AGC AAG TCA TTG AGC       602
   Ile Ser Gln Asp Gly Ser Asp Gln Ala Val Lys Ser Lys Ser Leu Ser
                   145                 150                 155
   TAT AAT CAA TTA ACA TAT ATG CAG CAC TTG GAT TTT GAA CCA GTG GTC       650
   Tyr Asn Gln Leu Thr Tyr Met Gln His Leu Asp Phe Glu Pro Val Val
               160                 165                 170
   ACT GAA AGG CCT GGT GAA CTG ACT GCG TAC TAC AAG ATT GCA CGT CAC       698
   Thr Glu Arg Pro Gly Glu Leu Thr Ala Tyr Tyr Lys Ile Ala Arg His
           175                 180                 185
   TAC AAG TGG GCA CTG GAC CAG TTG TTT TAC AAA CAC AAA TTT AGT CGA       746
   Tyr Lys Trp Ala Leu Asp Gln Leu Phe Tyr Lys His Lys Phe Ser Arg
       190                 195                 200
   GTG ATT ATA CTA GAA GAC GAT ATG GAA ATT GCT CCA GAC TTC TTT GAT       794
   Val Ile Ile Leu Glu Asp Asp Met Glu Ile Ala Pro Asp Phe Phe Asp
   205                 210                 215                 220
   TAC TTT GAG GCT GCA GCT AGT CTC ATG GAT AGG GAT AAA ACC ATT ATG       842
   Tyr Phe Glu Ala Ala Ala Ser Leu Met Asp Arg Asp Lys Thr Ile Met
                   225                 230                 235
   GCT GCT TCA TCA TGG AAT GAT AAT GGA CAG AAG CAG TTT GTG CAT GAT       890
   Ala Ala Ser Ser Trp Asn Asp Asn Gly Gln Lys Gln Phe Val His Asp
               240                 245                 250
   CCC TAT GCG CTA TAC CGA TCA GAT TTT TTT CCT GGC CTT GGG TGG ATG       938
   Pro Tyr Ala Leu Tyr Arg Ser Asp Phe Phe Pro Gly Leu Gly Trp Met
           255                 260                 265
   CTC AAG AGA TCG ACT TGG GAT GAG TTA TCA CCA AAG TGG CCA AAG GCT       986
   Leu Lys Arg Ser Thr Trp Asp Glu Leu Ser Pro Lys Trp Pro Lys Ala
       270                 275                 280
   TAC TGG GAT GAT TGG CTG AGA CTA AAG GAA AAC CAT AAA GGC CGC CAA      1034
   Tyr Trp Asp Asp Trp Leu Arg Leu Lys Glu Asn His Lys Gly Arg Gln
   285                 290                 295                 300
   TTC ATT GCA CCG GAA GTC TGT AGA ACA TAC AAT TTT GGT GAA CAT GGG      1082
   Phe Ile Ala Pro Glu Val Cys Arg Thr Tyr Asn Phe Gly Glu His Gly
                   305                 310                 315
   TCT AGT TTG GGA CAG TTT TTC AGT CAG TAT CTG GAA CCT ATA AAG CTA      1130
   Ser Ser Leu Gly Gln Phe Phe Ser Gln Tyr Leu Glu Pro Ile Lys Leu
               320                 325                 330
   AAC GAT GTG ACG GTT GAC TGG AAA GCA AAG GAC CTG GGA TAC CTG ACA      1178
   Asn Asp Val Thr Val Asp Trp Lys Ala Lys Asp Leu Gly Tyr Leu Thr
           335                 340                 345
   GAG GGA AAC TAT ACC AAG TAC TTT TCT GGC TTA GTG AGA CAA GCA CGA      1226
   Glu Gly Asn Tyr Thr Lys Tyr Phe Ser Gly Leu Val Arg Gln Ala Arg
       350                 355                 360
   CCA ATT CAA GGT TCT GAC CTT GTC TTA AAG GCT CAA AAC ATA AAG GAT      1274
   Pro Ile Gln Gly Ser Asp Leu Val Leu Lys Ala Gln Asn Ile Lys Asp
   365                 370                 375                 380
   GAT GAT CGT ATC CGG TAT AAA GAC CAA GTA GAG TTT GAA CGC ATT GCA      1322
   Asp Asp Arg Ile Arg Tyr Lys Asp Gln Val Glu Phe Glu Arg Ile Ala
                   385                 390                 395
   GGG GAA TTT GGT ATA TTT GAA GAA TGG AAG GAT GGT GTG CCA CGA ACA        1370
   Gly Glu Phe Gly Ile Phe Glu Glu Trp Lys Asp Gly Val Pro Arg Thr
               400                 405                 410
   GCA TAT AAA GGA GTA GTG GTG TTT CGA ATC CAG ACA ACA AGA CGT GTA        1418
   Ala Tyr Lys Gly Val Val Val Phe Arg Ile Gln Thr Thr Arg Arg Val
           415                 420                 425
   TTC CTG GTT GGG CCA GAT TCT GTA ATG CAG CTT GGA ATT CGA AAT TCC        1466
   Phe Leu Val Gly Pro Asp Ser Val Met Gln Leu Gly Ile Arg Asn Ser
       430                 435                 440
   TGA TGCAAAACAT ATGAAAGGAA AAGAAGATTT TGGACCGCAT GCAGCCTCCT             1519
    *
   445
   TCTAGCAGCT GTTAGGTTGT ATTGTTATTT ATGGATGAGT TTGTAGAGCG GTGGGGTTAA      1579
   CTTTAACAGC AAGGAAGCTC TGGTGACCAG GCTGATTGGC TTAGAAGTTA TGGGAACCCC      1639
   TTGAAAGGGT CAGGGTTAAA TATATTTCAG TTGTTTTATT AGTGATTATC TTGTGGGTAA      1699
   CTTATACGAA TGCAAATCAT TCTATGCAGT TTTTCTTCGT CCCACTTGTT TTGGCTTCTC      1759
   TATTGCTAGT GTACATATCT CTTCAAACAT GTACTAAATA ATGCGTGTTG CTTCAAAGAA      1819
   GTAACTTTTA TTAAAAAAAA AAAAAAAAAC TCGAG                                 1854
(2) ANGABEN ZU SEQ ID NO: 6:
   (i) SEQUENZKENNZEICHEN:
      (A) LÄNGE: 445 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 6:
   Met Ala Arg Ile Ser Cys Asp Leu Arg Phe Leu Leu Ile Pro Ala Ala
    1               5                  10                  15
   Phe Met Phe Ile Tyr Ile Gln Met Arg Leu Phe Gln Thr Gln Ser Gln
               20                  25                  30
   Tyr Ala Asp Arg Leu Ser Ser Ala Ile Glu Ser Glu Asn His Cys Thr
           35                  40                  45
   Ser Gln Met Arg Gly Leu Ile Asp Glu Val Ser Ile Lys Gln Ser Arg
       50                  55                  60
   Ile Val Ala Leu Glu Asp Met Lys Asn Arg Gln Asp Glu Glu Leu Val
   65                  70                  75                  80
   Gln Leu Lys Asp Leu Ile Gln Thr Phe Glu Lys Lys Gly Ile Ala Lys
                   85                  90                  95
   Leu Thr Gln Gly Gly Gln Met Pro Val Ala Ala Val Val Val Met Ala
               100                 105                 110
   Cys Ser Arg Ala Asp Tyr Leu Glu Arg Thr Val Lys Ser Val Leu Thr
   115 120 125
   Tyr Gln Thr Pro Val Ala Ser Lys Tyr Pro Leu Phe Ile Ser Gln Asp
   130 135 140
   Gly Ser Asp Gln Ala Val Lys Ser Lys Ser Leu Ser Tyr Asn Gln Leu
   145 150 155 160
   Thr Tyr Met Gln His Leu Asp Phe Glu Pro Val Val Thr Glu Arg Pro
   165 170 175
   Gly Glu Leu Thr Ala Tyr Tyr Lys Ile Ala Arg His Tyr Lys Trp Ala
   180 185 190
   Leu Asp Gln Leu Phe Tyr Lys His Lys Phe Ser Arg Val Ile Ile Leu
   195 200 205
   Glu Asp Asp Met Glu Ile Ala Pro Asp Phe Phe Asp Tyr Phe Glu Ala
   210 215 220
   Ala Ala Ser Leu Met Asp Arg Asp Lys Thr Ile Met Ala Ala Ser Ser
   225 230 235 240
   Trp Asn Asp Asn Gly Gln Lys Gln Phe Val His Asp Pro Tyr Ala Leu
   245 250 255
   Tyr Arg Ser Asp Phe Phe Pro Gly Leu Gly Trp Met Leu Lys Arg Ser
   260 265 270
   Thr Trp Asp Glu Leu Ser Pro Lys Trp Pro Lys Ala Tyr Trp Asp Asp
   275 280 285
   Trp Leu Arg Leu Lys Glu Asn His Lys Gly Arg Gln Phe Ile Ala Pro
   290 295 300
   Glu Val Cys Arg Thr Tyr Asn Phe Gly Glu His Gly Ser Ser Leu Gly
   305 310 315 320
   Gln Phe Phe Ser Gln Tyr Leu Glu Pro Ile Lys Leu Asn Asp Val Thr
   325 330 335
   Val Asp Trp Lys Ala Lys Asp Leu Gly Tyr Leu Thr Glu Gly Asn Tyr
   340 345 350
   Thr Lys Tyr Phe Ser Gly Leu Val Arg Gln Ala Arg Pro Ile Gln Gly
   355 360 365
   Ser Asp Leu Val Leu Lys Ala Gln Asn Ile Lys Asp Asp Asp Arg Ile
   370 375 380
   Arg Tyr Lys Asp Gln Val Glu Phe Glu Arg Ile Ala Gly Glu Phe Gly
   385 390 395 400
   Ile Phe Glu Glu Trp Lys Asp Gly Val Pro Arg Thr Ala Tyr Lys Gly
   405 410 415
   Val Val Val Phe Arg Ile Gln Thr Thr Arg Arg Val Phe Leu Val Gly
   420 425 430
   Pro Asp Ser Val Met Gln Leu Gly Ile Arg Asn Ser  *
           435                 440                445

## Beschreibung

Die Erfindung betrifft pflanzliche *GntI*-Sequenzen, insbesondere pflanzliche Nukleinsäuresequenzen, die das Enzym N-Acetylglucosaminyltransferase I (GnTI) kodieren, wie auch davon abgeleitete *GntI*-"antisense"- bzw. "sense"-Konstrukte, und deren Translationsprodukte, gegen diese Translationsprodukte gerichtete Antikörper sowie die Verwendung der Sequenzinformation zur Gewinnung von transformierten Mikroorganismen und von transgenen Pflanzen, einschließlich solchen mit verminderter oder fehlender N-Acetylglucosaminyltransferase I-Aktivität. Derartige Pflanzen mit verminderter oder fehlender N-Acetylglucosaminyltransferase I-Aktivität sind zur Herstellung von Glykoproteinen spezifischer Konstitution bezüglich der Zuckerreste von großer Bedeutung.

### Stand der Technik:

In Eukaryonten werden Glykoproteine im Endoplasmatischen Retikulum (ER) cotranslational (d.h. bei Import in das ER-Lumen) durch Verknüpfung von zunächst membrangebundenen Glykanen (an Dolicholpyrophosphat) mit spezifischen Asparagin-Resten in der wachsenden Polypeptidkette zusammengesetzt (N-Glykosylierung). In höheren Organismen unterliegen Zuckereinheiten, die an der Oberfläche der gefalteten Polypeptidkette liegen, in den Golgi-Zisternen weiteren Trimm- und Modifikationsreaktionen (Ref. 1). Durch verschiedene Glykosidasen und Glykosyltransferasen im ER werden zunächst typische Glc₃Man₉GlcNAc₂-Grundeinheiten des "high"-Mannose-Typs gebildet und anschließend bei Passage durch die verschiedenen Golgi-Zisternen in sogenannte "komplexe" Glykane umgewandelt. Letztere zeichnen sich durch weniger Mannose-Einheiten und den Besitz weiterer Zuckerreste, wie Fucose, Galaktose und/oder Xylose in Pflanzen bzw. Sialinsäure (N-Acetylneuraminsäure, NeuNAc) in Säugern, aus (Ref. 1,2,3). Das Ausmaß der Modifikationen ist von Glykoprotein zu Glykoprotein verschieden. Einzelne Polypeptidketten können heterogene Zuckerketten tragen. Zudem kann das Glykosylierungsmuster für ein bestimmtes Polypeptid variieren (gewebespezifische Unterschiede), und muß auch nicht immer bezüglich einer bestimmten Glykosylierungsstelle gleich sein, was als "Mikroheterogenität" bezeichnet wird (Ref. 4.5). Die Rolle von Asparagin-ständigen Glykanen ist bislang kaum verstanden, was u.a. daraus resultiert, daß diese mehrere Funktionen erfüllen können (Ref. 6). Jedoch ist anzunehmen, daß beispielsweise der Schutz einer Polypeptidkette vor proteolytischem Abbau auch durch Glykane eines einfacheren Oligomannosyl-Typs gewährleistet werden kann (Ref. 7) .

### Problemstellung:

Glykoproteine sind für Medizin und Forschung von großer Bedeutung. Eine Isolierung von Glykoproteinen im Großmaßstab ist jedoch aufwendig und teuer. Die direkte Anwendung konventionell isolierter Glykoproteine ist oft problematisch, da einzelne Reste der Glykan-Komponenten bei Verabreichung als Therapeutikum ungewünschte Nebenwirkungen auslösen können. Dabei trägt die Glykan-Komponente vor allem zu den physikochemischen Eigenschaften (wie Faltung, Stabilität und Löslichkeit) der Glykoproteine bei. Des weiteren tragen isolierte Glykoproteine, wie bereits ausgeführt, selten einheitliche Zuckerreste, was als "Mikroheterogenität" bezeichnet wird.

Hefen erweisen sich zur Gewinnung von Glykoproteinen für Medizin und Forschung als ungeeignet, da sie nur Glykosylierungen zum sogenannten "high"-Mannose-Typ durchführen können. Insekten und höhere Pflanzen zeigen zwar "komplexe", aber von Tieren abweichende Glykoproteinmodifikationen. Aus Pflanzen isolierte Glykoproteine wirken deshalb in Säugern stark antigen. Tierische Organismen mit Glykosylierungsdefekten sind meist nicht lebensfähig, da die terminalen Glykan-Reste (beispielsweise membranständiger Glykoproteine) meist biologische Signalfunktion besitzen und vor allem für die Zell-Zellerkennung während der Embryonalentwicklung unentbehrlich sind. Es existieren zwar bereits Säugerzellinien mit definierten Glykosylierungsdefekten, jedoch ist deren Kultivierung arbeitsintensiv und teuer.

Im Einzelnen wurden für Säuger auf Zellkulturebene bereits unterschiedliche Glykosylierungsmutanten beschrieben (Ref. 7,8, 9,10). Diese Mutanten sind entweder in der Biosynthese reifer Oligosaccharidketten am Dolicholpyrophosphat oder in der Glykan-Prozessierung betroffen bzw. zeigen Abweichungen in ihren terminalen Zuckerresten. Einige dieser Zellinien weisen einen konditional-lethalen Phänotyp auf oder zeigen Defekte im intrazellulären Proteintransport. Die Folgen dieser Defekte für den intakten Organismus sind schwer abschätzbar. Es wurde beobachtet, daß eine Veränderung im Muster komplexer Glykane auf Zelloberflächen von Säugern mit Tumor- und Metastasenbildung einhergeht, obwohl eine funktionale Beziehung noch nicht eindeutig nachgewiesen werden konnte (Ref. 9). Glykosylierungsmutanten, kommen in Säugern daher sehr selten vor. Diese unter HEMPAS (Hereditary Erythroblastic Multinuclearity with a Positive Acidified Serum lysis test) zusammengefaßten Defekte (Ref. 10,11) beruhen entweder auf einem Defizit von Mannosidase II und/oder niedrigen Gehalten des Enzyms N-Acetylglucosaminyltransferase II (GnTII) und wirken sich stark einschränkend auf die Lebensfähigkeit des mutierten Organismus aus. *GntI*-"knock out"-Mäuse, in denen das Gen für GnTI zerstört wurde, sterben bereits "in utero" an multiplen Entwicklungsdefekten (mündliche Mitteilung, H. Schachter, Toronto).

Für Pflanzen waren bis vor kurzem keine vergleichbaren Mutanten bekannt. Durch den Einsatz eines Antiserums, das spezifisch "komplex"-modifizierte Glykanketten pflanzlicher Glykoproteine erkennt und hauptsächlich gegen die hoch-antigenen β1→2 verknüpften Xylose-Reste gerichtet ist (Ref. 12), konnte die Anmelderin aus einer EMS-mutagenisierten F2-Population der genetischen Modellpflanze *Arabidopsis thaliana* mehrere unabhängige Mutanten isolieren, die keine "komplexe" Glykoproteinmodifikation mehr zeigten (complex glycan, *cgl-*Mutanten). Nach mindestens fünf Rückkreuzungen, jeweils gefolgt von intermittierenden Selbstungen (zum Wiederauffinden des rezessiven Defekts), wurden die Glykoproteine analysiert. Diese trugen hauptsächlich Glykane des Man₅GlcNAc₂-Typs, was auf einen Defekt in N-Acetylglucosaminyltransferase I (GnTI) hindeutete (Ref. 8). Tatsächlich fehlte den *Arabidopsis cgl*-Mutanten GnTI-Aktivität (Ref. 13), welche normalerweise die erste Reaktion im Syntheseweg zu "komplex" modifizierten Zuckerketten katalysiert (Ref. 1). Nach bisherigen Beobachtungen resultiert daraus allerdings keine Beeinträchtigung der Lebensfähigkeit der mutierten Pflanzen. In neueren Publikationen werden Pflanzen als mögliche Quelle zur Herstellung von pharmazeutisch relevanten Glykoproteinen oder Vakzinen vorgeschlagen (Ref. 14,15). Darin wird jedoch übersehen, daß aus Pflanzen isolierte Glykoproteine in Säugern starke Immunreaktionen auslösen können, was bisher einer Produktion heterologer Glykoproteine in Kulturpflanzen im Wege stand.

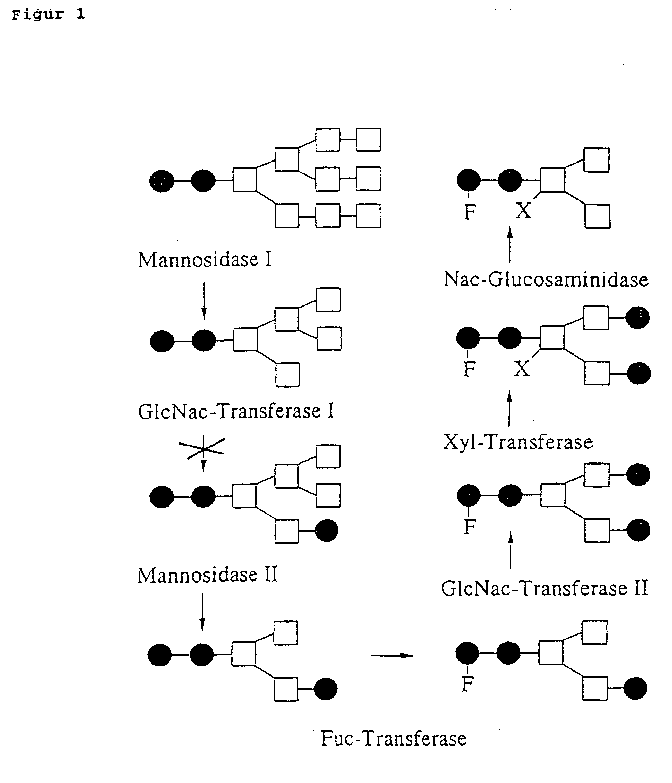

Pflanzen kommen weitgehend ohne "komplex" modifizierte Glykoproteine aus, wie die Anmelderin am Beispiel der *Arabidopsis cgl*-Mutante zeigen konnte (Ref. 13). In der Mutante werden sekretorische Proteine im ER zunächst normal glykosyliert. Im Golgi-Apparat der *cgl*-Mutante bleiben die über Asparagin-Reste (N-Glykosylierung) an das Polypeptidrückgrat gebundenen Oligomannosylketten dann jedoch auf Stufe von Man₅GlcNAc₂-Resten stehen, da N-Acetylglucosaminyltransferase I (GnTI)-Aktivität fehlt (Fig. 1). Durch diesen Biosyntheseblock wird die pflanzenspezifische "komplexe" Glykoproteinmodifikation und insbesonders die Anheftung von α1→3 Fukose- und β1→2 Xylose-Resten verhindert, wodurch die stark antigene Wirkung auf den Säugerorganismus entfällt. Als krautige Pflanze besitzt Arabidopsis jedoch wenig verwertbare Biomasse. Zur Herstellung von biotechnologisch relevanten Glykoproteinen im Großmaßstab sind diese *cgl*-Pflanzen deshalb weniger geeignet. Als Alternative wären Kultursorten, insbesondere Solanaceen, wie z.B. Kartoffel, Tabak, Tomate oder Paprika, und des weiteren Luzerne, Raps, Rüben, Soja, Salat, Mais, Reis, und Getreide, mit fehlender oder stark gedrosselter GnTI-Aktivität ideal zur Produktion von heterologen Glykoproteinen in Pflanzen. Dazu würden sich Verfahren des "homology-dependent gene silencing" (Ref. 16,17) anbieten.

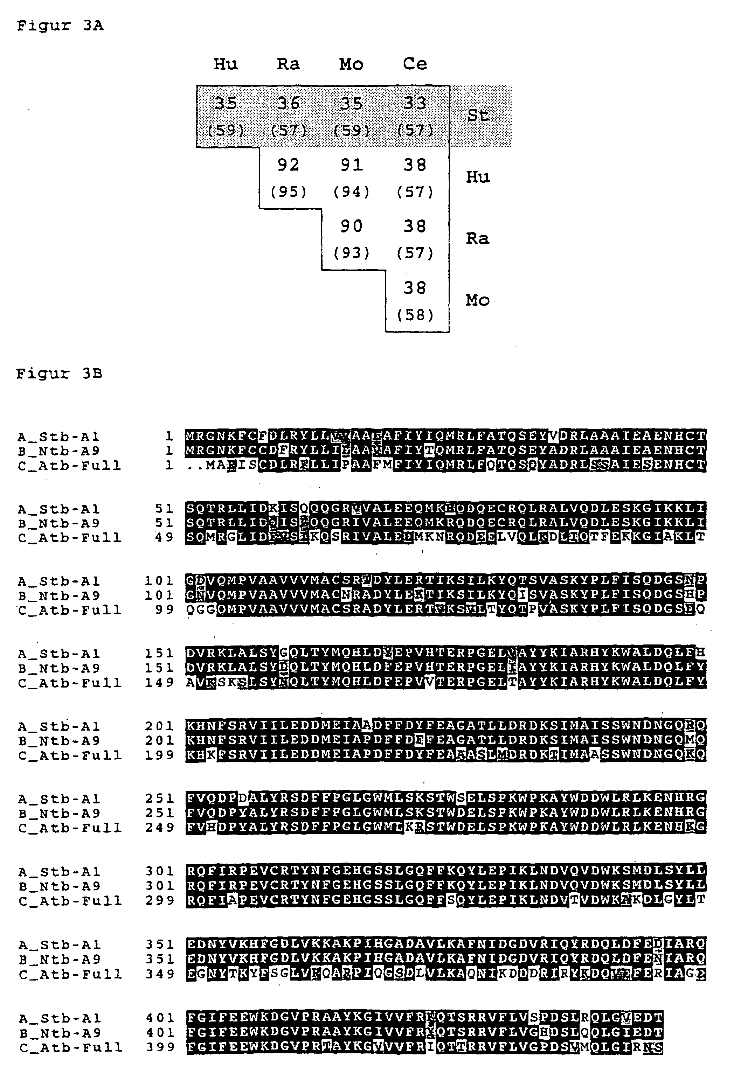

Wie Fig. 3 zeigt, ist die Homologie der ersten ermittelten pflanzlichen *GntI*-Sequenz aus Kartoffel (*Solanum tuberosum* L., St) im Vergleich mit den entsprechenden bekannten Sequenzen aus tierischen Organismen außerordentlich niedrig (nur 30-40% Identität auf Proteinebene, vgl. Fig. 3A), so daß eine effiziente Drosselung der endogenen "komplexen" Glykoproteinmodifikation in Pflanzen mittels "antisense" bzw. "sense"-Suppression (Ref. 21) durch die Verwendung bereits bekannter heterologer *GntI*-Gen-sequenzen mit hoher Wahrscheinlichkeit nicht erreicht werden kann.

Für Medizin und Forschung besteht daher nach wie vor ein Bedarf, rekombinante Glykoproteine mit minimalen und einheitlichen, also definierten Zuckerresten in geeigneten Organismen kostengünstig herstellen zu können.

### Wesen der Erfindung:

Nachdem die Anmelderin erstmals pflanzliche *GntI*-cDNA-Sequenzen isolieren und aufklären konnte, ist es nun u.a. möglich, beliebige Pflanzen mit gedrosselter oder fehlender GnTI-Aktivität zu gewinnen, insbesondere herzustellen, bzw. entsprechende Mutanten durch revers-genetische Ansätze nach Transposon-(Ref. 18) bzw. T-DNA-Insertion (Ref. 19) aufzuspüren, um in diesen dann Glykoproteine mit niedrigem Antigenpotential zu produzieren.

### i) Enzyme:

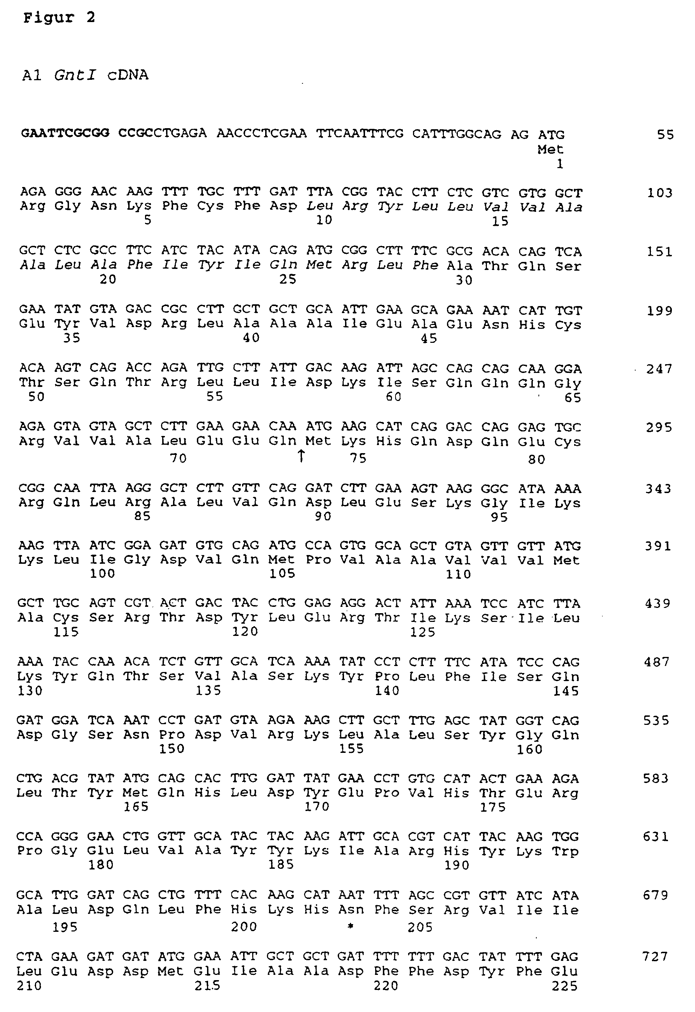

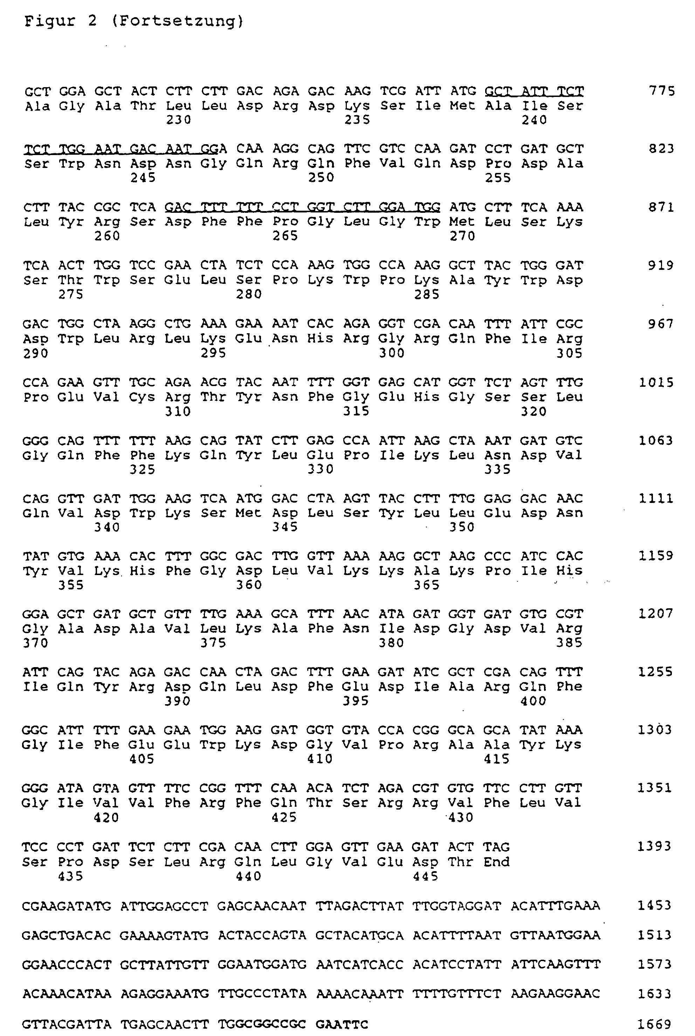

Die Erfindung umfaßt allgemein verschiedene N-Acetylglucosaminyltransferase I-Enzyme (EC 2.4.1.101) aus Pflanzen, z.B. aus Kartoffel (*Solanum tuberosum* L.), Tabak (*Nicotiana tabacum* L.) und *Arabidopsis thaliana.* Insbesondere betrifft die Erfindung die Enzyme, die die in Fig. 2 und 3B sowie in dem beigefügten Sequenzprotokoll angegebenen Aminosäuresequenzen aufweisen oder enthalten.

Von der Erfindung umfaßt sind ferner von den Aminosäuresequenzen der genannten Enzyme durch Aminosäuresubstitution, -deletion, -insertion, -modifikation oder durch C- und/oder N-terminale Verkürzung und/oder Verlängerung abgeleitete Enzyme, die, sofern sie enzymatische Aktivität zeigen, eine dem Ausgangsenzym vergleichbare Spezifität, also N-Acetylglucosaminyltransferase I-Aktivität, sowie ggf. vergleichbare Aktivität aufweisen.

Unter einer vergleichbaren Aktivität wird im vorliegenden Zusammenhang eine Aktivität verstanden, die bis zu 100% über oder unter der Aktivität des Ausgangsenzyms liegt. Von der Erfindung sind dementsprechend auch abgeleitete Enzyme oder Proteine mit sehr geringer oder vollständig fehlender enzymatischer Aktivität, nachweisbar mittels eines oder mehrerer der nachfolgend angegebenen Tests, umfaßt. Zum Nachweis der Enzymaktivität dient ein Standard-Test, der mit Mikrosomen-Fraktionen entweder radioaktiv, z.B. mit UDP-[6-³H]GlcNac als Substrat (Ref. 13), oder nicht-radioaktiv (HPLC-Methode; Ref. 20) durchgeführt wird. Pflanzliche GnTI-Aktivität kann subzellulär in Golgi-Fraktionen nachgewiesen werden (Ref. 21). Enzymanreicherungen aus Pflanzen sind aufgrund der geringen Ausbeuten jedoch nahezu unmöglich.

Gegebenenfalls alternativ kann ein erfindungsgemäßes abgeleitetes Enzym als ein Enzym definiert werden, für das eine das Enzym kodierende DNA-Sequenz ermittelt oder abgeleitet werden kann, die mit der das Ausgangsenzym kodierenden DNA-Sequenz bzw. der dazu komplementären Sequenz unter stringenten Bedingungen, wie sie nachfolgend definiert werden, hybridisiert.

Ein dergestalt abgeleitetes Enzym stellt beispielsweise eine Isoform dar, die die Aminosäuren 74 bis 446 der in Fig. 2 und SEQ ID NO: 1 und 2 angegebenen Aminosäuresequenz umfaßt. Dieser Isoform fehlt u.a. der durch die Aminosäuren 10 bis 29 gebildete Membrananker, so daß diese Enzym-Isoform möglicherweise aufgrunddessen im Cytosol der Pflanze lokalisiert ist.

Als Beispiele für C- und/oder N-terminal verlängerte Proteine können Fusionsproteine genannt werden, die neben einer erfindungsgemäßen Aminosäuresequenz ein weiteres Protein umfassen, das beispielsweise eine andersartige enzymatische Aktivität aufweist oder auf andere Weise, z.B. aufgrund von Fluoreszenz oder Phosphoreszenz oder aufgrund einer Reaktivität mit spezifischen Antikörpern oder durch Binden an entsprechende Affinitätsmatrices, leicht nachweisbar ist.

Die Erfindung umfaßt gleichfalls Fragmente der genannten Enzyme, die ggf. keine enzymatische Aktivität mehr aufweisen. Diese Fragmente zeigen in der Regel jedoch antigene Wirkung in einem damit immunisierten Wirt und können folglich als Antigen zur Erzeugung monoklonaler oder polyklonaler Antikörper durch Immunisierung eines Wirtes mit diesen eingesetzt werden.

Die Erfindung betrifft darüberhinaus auch N-Acetylglucosaminyltransferase 1-Enzyme insbesondere aus anderen Varietäten und Pflanzenarten, die zugänglich sind aufgrund der Hybridisierung ihrer Gene oder eines oder mehrerer Abschnitte ihrer Gene
- mit einer oder mehreren der nachfolgend erläuterten erfindungsgemäßen DNA-Sequenzen und/oder DNA-Fragmente und/oder
- mit geeigneten erfindungsgemäßen Hybridisierungssonden, die ausgehend von den im Sequenzprotokoll angegebenen Aminosäuresequenzen unter Berücksichtigung der Degeneration des genetischen Codes hergestellt werden können.

Von der Erfindung umfaßt sind ferner nach den vorstehend erläuterten Maßgaben von diesen N-Acetylglucosaminyltransferase I-Enzymen abgeleitete Enzyme oder Proteine, einschließlich Fusionsproteinen von diesen, sowie Fragmente aller dieser Enzyme oder Proteine.

### ii) Antikörper

Ein weiterer Aspekt der Erfindung betrifft die Verwendung der vorstehend genannten Aminosäuresequenzen bzw. von antigen wirksamen Fragmenten davon zur Erzeugung von monoklonalen oder polyklonalen Antikörpern oder -seren durch Immunisierung von Wirten mit diesen Aminosäuresequenzen bzw. Fragmenten sowie Antikörper bzw. -seren an sich, die die vorstehend erläuterten Enzyme und/oder Antigene spezifisch erkennen und binden. Die allgemeine Vorgehensweise und die entsprechenden Techniken zur Erzeugung polyklonaler und monoklonaler Antikörper sind dem Fachmann gleichfalls wohlbekannt.

Beispielhaft wurde rekombinantes GnTI-Protein aus *Solanum tuberosum* mit 10 N-terminalen Histidin-Resten ("His-tag") durch Verwendung eines Fragments der in Fig. 2 und SEQ ID NO: 1 dargestellten *GntI*-cDNA (Nukleotide 275 bis 1395) in *E. coli* überexprimiert und nach Affinitätsreinigung über eine Metall-Chelat-Matrix als Antigen zur Erzeugung von polyklonalen Antiseren in Kaninchen eingesetzt (vgl. Beispiele 5 und 6).

Eine Anwendungsmöglichkeit der erfindungsgemäßen Antikörper besteht für das "Screenen" von Pflanzen auf das Vorhandensein von N-Acetylglucosaminyltransferase I.

Eine Bindung des erfindungsgemäßen Antikörpers an pflanzliche(s) Protein(e) zeigt das Vorliegen eines mit diesem Antikörper nachweisbaren N-Acetylglucosaminyltransferase I-Enzyms an. In einem späteren Schritt kann dieser Antikörper darüberhinaus, in der Regel dann kovalent an einen Träger gebunden, gegebenenfalls auch zur Anreicherung oder Reinigung des Enzyms mittels Säulenchromatographie eingesetzt werden.

Ein negatives Bindungsergebnis mit dem erfindungsgemäßen Antikörper, d.h. fehlende Bindung an die pflanzlichen Proteine, läßt wiederum auf ein fehlendes (oder durch Mutation stark verändertes) N-Acetylglucosaminyltransferase I-Enzym und damit auf fehlende oder stark verminderte N-Acetylglucosaminyltransferase I-Aktivität in einer untersuchten Pflanze schließen.

Techniken zum Ausführen der vorstehend angesprochenen "Screening"-Tests oder zur Enzymanreicherung bzw. -reinigung unter Einsatz von Antikörpersäulen oder anderen Affinitätsmatrices (vgl. Beispiele 5 und 6) sind dem Fachmann wohlbekannt.

### iii) DNA-Sequenzen

Die Erfindung umfaßt ferner DNA-Sequenzen, die die erfindungsgemäßen Aminosäuresequenzen, einschließlich davon gemäß den vorstehenden Maßgaben abgeleitete Aminosäuresequenzen kodieren. Insbesondere betrifft die Erfindung das den in den Figuren 2 und 3B und im Sequenzprotokoll aufgeführten Aminosäuresequenzen jeweils zugrundeliegende Gen, und ganz besonders die in Fig. 2 und im Sequenzprotokoll aufgeführten cDNA-Sequenzen, sowie von diesen Genen und DNA-Sequenzen abgeleitete DNA-Sequenzen.

Unter abgeleiteten DNA-Sequenzen werden Sequenzen verstanden, die durch Substitution, Deletion und/oder Insertion von einzelnen oder mehreren und/oder kleineren Gruppen von Nukleotiden der vorstehend angegebenen Sequenzen und/oder durch Verkürzung oder Verlängerung am 5'- und/oder 3'-Ende erhalten werden. Die Modifizierungen innerhalb der DNA-Sequenz können zu abgeleiteten DNA-Sequenzen führen, die identische Aminosäuresequenzen verglichen mit der von der Ausgangs-DNA-Sequenz kodierten Aminosäuresequenz kodieren, oder aber auch zu solchen, bei denen einzelne oder einige wenige Aminosäuren gegenüber der Aminosäuresequenz, die die Ausgangs-DNA-Sequenz kodiert, verändert, d.h. substituiert, deletiert und/oder insertiert sind, oder auch solchen, die - ggf. zusätzlich - C- und/oder N-terminal verkürzt und/oder verlängert sind.

Die Erfindung erstreckt sich gleichfalls auf die zu den erfindungsgemäßen Genen und DNA-Sequenzen komplementären Sequenzen sowie auf deren RNA-Transkriptionsprodukte.

Von der Erfindung umfaßt sind insbesondere sämtliche, nach den vorstehend angegebenen Maßgaben abgeleiteten Sequenzen, die unter stringenten Bedingungen mit den oben erläuterten Ausgangssequenzen oder den dazu komplementären Sequenzen oder Teilen davon hybridisieren, wie auch DNA-Sequenzen, die derartige Sequenzen umfassen.

Als Hybridisierung unter stringenten Bedingungen im Sinne der Erfindung wird eine Hybridisierung bei Vorgehensweise gemäß einem oder mehreren der nachstehend angegebenen Verfahren verstanden. Hybridisieren: Bis zu 20 Std. in PEG-Puffer nach Church und Gilbert (0,25 M Na₂HPO₄, 1 mM EDTA, 1% (w/v) BSA, 7% (w/v) SDS, pH 7,5 mit Phosphorsäure; Ref. 22) bei 42°C oder in Standard-Hybridisierungspuffern mit Formamid bei 42°C oder ohne Formamid bei 68°C (Ref. 23). Waschen: 3-mal 30 min bei 65°C in 3-fach SSC-Puffer (Ref. 23), 0,1% SDS.

Im Rahmen der vorliegenden Anmeldung ist der Begriff "Hybridisierung" stets als Hybridisierung unter stringenten Bedingungen, wie vorstehend angegeben, zu verstehen, auch wenn dies im Einzelfall nicht explizit angegeben ist.

Darüberhinaus erstreckt sich die Erfindung auch auf Fragmente der vorstehend erläuterten DNA-Sequenzen, einschließlich der nach den vorstehenden Maßgaben abgeleiteten DNA-Sequenzen, auf von derartigen Fragmenten durch Nukleinsäuresubstitution, -insertion und/oder -deletion abgeleitete Fragmente sowie auf die entsprechenden Fragmente mit dazu komplementären Sequenzen. Derartige Fragmente sind u.a. als Sequenzierungs- oder PCR-Primer, "Screening"-Sonden und/oder für Verwendungen, wie sie nachfolgend erläutert werden, geeignet. Für eine Verwendung als "Screening"- oder Hybridisierungssonde werden die erfindungsgemäßen DNA-Fragmente häufig radioaktiv markiert eingesetzt. Fragmente mit Sequenzen, die von den vorstehend definierten Ausgangssequenzen durch Substitution, Deletion und/oder Insertion von einem oder mehreren Nukleotiden abgeleitet sind, bzw. die dazu komplementären Sequenzen sind in dem Umfange von der Erfindung umfaßt, als sie unter den vorstehend angegebenen stringenten Bedingungen mit den Ausgangssequenzen bzw. den dazu komplementären Sequenzen hybridisieren.

Erfindungsgemäße DNA-Fragmente können beispielsweise auf der Grundlage der im Sequenzprotokoll und in Figur 2 angegebenen DNA-Sequenzen ausgehend von pflanzlicher DNA mittels Restriktionsendonukleasen unter Nutzung geeigneter Restriktionsschnittstellen oder durch Einsatz von PCR mittels geeignet synthetisierter Primer erhalten oder alternativ auch chemisch synthetisiert werden. Derartige Techniken sind dem Fachmann wohlbekannt.

Die Erfindung betrifft darüberhinaus auch jegliche DNA-Sequenzen, die ein Gen darstellen oder Teil eines Gens sind, das das Enzym N-Acetylglucosaminyltransferase I kodiert, und die in ihrer Gesamtheit oder in einem Teilabschnitt
- mit einer der erfindungsgemäßen DNA-Sequenzen und/oder
- mit einem oder mehreren der erfindungsgemäßen DNA-Fragmente und/oder
- mit einer DNA-Sequenz, die von den im Sequenzprotokoll angegebenen Aminosäuresequenzen unter Berücksichtigung der Degeneration des genetischen Codes abgeleitet worden ist,
unter stringenten Bedingungen hybridisieren.

Als DNA-Fragmente werden hierzu Hybridisierungs- oder "Screening"-Sonden eingesetzt, die üblicherweise mindestens 15 Nukleotide, typischerweise zwischen 15 und 30 Nukleotide, gegebenenfalls aber auch wesentlich mehr, umfassen. Es können dafür beispielsweise die in Beispiel 1 eingesetzten Primer Verwendung finden. Alternativ können DNA-Sequenzen geeigneter Länge, abgeleitet von den im Sequenzprotokoll angegebenen DNA-Sequenzen, eingesetzt werden. Als dritte Möglichkeit können geeignete erfindungsgemäße Hybridisierungssonden ausgehend von den im Sequenzprotokoll angegebenen Aminosäuresequenzen unter Berücksichtigung der Degeneration des genetischen Codes entwickelt werden.

In diesem Sinne sind Gegenstand der Erfindung auch N-Acetylglucosaminyltransferase I kodierende Gene, die insbesondere aus anderen Varietäten oder Pflanzenarten aufgrund ihrer Hybridisierung mit den vorstehend angegebenen Hybridisierungssonden aufgefunden werden können, sowie davon gemäß den vorstehend erläuterten Maßgaben abgeleitete DNA-Sequenzen, DNA-Fragmente und -Konstrukte.

Die Isolierung des jeweiligen Gens und die Sequenzierung desselben nach der Auffindung mittels der erfindungsgemäßen Hybridisierungssonden liegen im Bereich der Fähigkeiten eines Fachmanns auf diesem Gebiet und sind exemplarisch in den Beispielen für N-Acetylglucosaminyltransferase I aus *Solanum tuberosum* und die entsprechenden Enzyme aus *Nicotiana tabacum* und Arabidopsis *thaliana* erläutert.

Gegenstand der Erfindung sind schließlich auch "antisense"-Sequenzen bezüglich jeglichen vorstehend erläuterten DNA-Sequenzen.

### iv) Konstrukte

Von der Erfindung werden auch Konstrukte umfaßt, die ggf. neben zusätzlichen 5'- und/oder 3'-Sequenzen, z.B. Linkern und/oder regulatorischen DNA-Sequenzen, oder andersartigen Modifikationen die erfindungsgemäßen DNA-Sequenzen, einschließlich der wie vorstehend ausgeführt abgeleiteten DNA-Sequenzen, umfassen.

Ein Beispiel hierfür sind Hybridisierungs- oder "Screening"-Sonden, die zusätzlich zu einer erfindungsgemäßen DNA-Sequenz noch ein in diesem Fall meist nichtradioaktives Nachweismittel für die Detektion von Hybridisierungsprodukten umfassen, z.B. fluoreszierende oder phosphoreszierende Moleküle, Biotin, Biotinderivate, Digoxigenin und Digoxigeninderivate. Es kommen in diesem Zusammenhang jedoch auch radioaktive oder nichtradioaktive Nachweismittel, die z.B. durch Endmarkierung an die erfindungsgemäße DNA-Sequenz angeheftet werden können, in Betracht.

Gegenstand der Erfindung sind auch "antisense"- und "sense"-Konstrukte bezüglich der erfindungsgemäßen DNA-Sequenzen und -Fragmente, nämlich bezüglich
- der im Sequenzprotokoll angegebenen DNA-Sequenzen und der zugrundeliegenden Gene,
- der davon nach den vorstehenden Maßgaben abgeleiteten DNA-Sequenzen,
- eines oder mehrerer Abschnitte dieser DNA-Sequenzen,
- DNA-Sequenzen insbesondere aus anderen Varietäten oder Pflanzenarten, die ein Gen darstellen oder Teil eines Gens sind, welches das Enzym N-Acetylglucosaminyltransferase I kodiert, und die
   -- mit einer der vorstehend genannten DNA-Sequenzen und/oder
   -- mit einem oder mehreren der vorstehend genannten DNA-Fragmente und/oder
   -- mit einer DNA-Sequenz, die von den im Sequenzprotokoll angegebenen Aminosäuresequenzen unter Berücksichtigung der Degeneration des genetischen Codes abgeleitet worden ist,
unter stringenten Bedingungen hybridisieren.

Des weiteren erstreckt sich die Erfindung auch auf jegliche DNA-Übertragungsysteme, wie Vektoren, Plasmide, Viren- und Phagengenome oder Cosmide, die die erfindungsgemäßen DNA-Sequenzen, z.B. das GntI-Gen, erfindungsgemäße cDNA und DNA-Abschnitte, wie sie im Sequenzprotokoll angegeben sind, Fragmente davon, insbesondere "antisense"- oder "sense"-Konstrukte und/oder davon nach den vorstehenden Maßgaben abgeleitete DNA-Sequenzen, enthalten.

Diverse Techniken zur Gewinnung oder Synthese erfindungsgemäßer DNA, DNA-Fragmente, Konstrukte und Übertragungssysteme, z.B. ausgehend von pflanzlicher DNA durch Restriktion mittels Restriktionsendonukleasen, PCR-Amplifizierung unter Einsatz geeigneter Primer, ggf. gefolgt von Klonierung und zusätzlicher chemischer oder enzymatischer Modifizierung, sind dem Fachmann wohlbekannt.

Eine Anwendungsmöglichkeit von erfindungsgemäßen DNA-Hybridisierungssonden liegt im Nachweis von N-Acetylglucosaminyltransferase I-Genen in anderen Pflanzen als jenen, aus denen die im Sequenzprotokoll angegebenen DNA-Sequenzen erhalten wurden, oder im Nachweis von möglichen (weiteren) Isoformen des N-Acetylglucosaminyltransferase I-Gens in den Ausgangspflanzen *Solanum tuberosum, Nicotiana tabacum* und *Arabidopsis thaliana.*

Kann für das Hybridisierungsexperiment auf eine genomische Bank oder cDNA-Bank einer Pflanze zurückgegriffen werden, liefert ein positives Hybridisierungsergebnis bei einem derartigen "Screening" oder Durchmustern der jeweiligen Bank den Hinweis auf einen Klon oder einige wenige Klone, die die gesuchte Sequenz, das N-Acetylglucosaminyltransferase I-Gen, vollständig oder teilweise in Verbindung mit nur einer begrenzten Menge weiterer DNA aus dem Genom der Zielpflanze enthalten, was die Klonierung und Sequenzierung des Zielgens entsprechend erleichtert. Alternativ kann ausgehend von pflanzlicher DNA und geeigneten Konstrukten, sogenannten PCR-Primern, auch eine PCR-Amplifikation des Gens oder von Teilen desselben vorgenommen werden, um Klonierung und Sequenzierung zu vereinfachen.

Ein Einsatz erfindungsgemäßer sequenzierungsprimer, die ausgehend von geeigneten Abschnitten der erfindungsgemäßen Sequenzen synthetisiert werden, ermöglicht z.B. eine genomische Sequenzierung ausgehend von der vollständigen, durch Restriktionsendonukleasen geschnittenen genomischen DNA einer Zielpflanze mittels der Church-Gilbert-Technik wie auch z.B. eine Sequenzierung auf cDNA-Ebene nach RT-PCR-Amplifikation der Gesamt-RNA der Zielpflanze (vgl. Bsp. 1).

Eine alternative Anwendungsmöglichkeit von erfindungsgemäßen DNA-Hybridisierungssonden, die von den im Sequenzprotokoll angegebenen DNA-Sequenzen abgeleitet sind, besteht in der erfindungsgemäßen Verwendung derselben zum Nachweis von Pflanzen mit verminderter oder fehlender N-Acetylglucosaminyltransferase I-Aktivität. Das Hybridisierungsexperiment dient zur Detektion des Gens der N-Acetylglucosaminyltransferase I (*GntI*) und erlaubt z.B. aufgrund eines negativen Hybridisierungsergebnisses unter stringenten Bedingungen den Rückschluß auf ein Fehlen des *GntI*-Gens und damit fehlende N-Acetylglucosaminyltransferase I-Aktivität in einer untersuchten Pflanze.

Derartige Hybridisierungstechniken zum Nachweis von Proteinen oder Genen insbesondere in Pflanzenmaterial mittels DNA-Sonden sind dem Fachmann ebenfalls geläufig. Es wird in diesem Zusammenhang auf die vorstehenden Ausführungen zu möglichen Hybridisierungsbedingungen unter Punkt iii) verwiesen. Geeignete DNA-Hybridisierungssonden umfassen in der Regel mindestens 15 Nukleotide mit einer Sequenz, die z.B. von den in Fig. 2 und im Sequenzprotokoll angegebenen cDNA-Sequenzen oder den entsprechenden *GntI*-Genen abgeleitet ist.

### v) Transformierte Mikroorganismen

Die Erfindung erstreckt sich des weiteren auf Mikroorganismen, wie z.B. Bakterien, Bakteriophagen, Viren, einzellige eukaryotische Organismen, wie Pilze, Hefen, Protozoen, Algen und humane, tierische und pflanzliche Zellen, die durch eine oder mehrere der erfindungsgemäßen DNA-Sequenzen oder eines oder mehrere der erfindungsgemäßen Konstrukte, wie vorstehend erläutert, transformiert wurden.

Verwendung finden erfindungsgemäße transformierte Mikroorganismen beispielsweise als Expressionssysteme für die transformierende Fremd-DNA zur Gewinnung der entsprechenden Expressionsprodukte. Typische Mikroorganismen für diese Zwecke sind Bakterien, wie beispielsweise *E*. *coli.* Des weiteren können erfindungsgemäße transformierte Mikroorganismen, insbesondere Agrobakterien, z.B. zur Transformation von Pflanzen unter Weitergabe der transformierenden Fremd-DNA eingesetzt werden.

Verfahren zur Transformation von Mikroorganismenzellen durch (Fremd-)DNA sind dem Fachmann wohlbekannt.

Hierfür werden z.B. als Expressionsvektoren bezeichnete Konstrukte eingesetzt, die die erfindungsgemäße DNA-Sequenz unter Kontrolle eines konstitutiven oder induzierbaren, ggf. zusätzlich gewebespezifischen Promotors enthalten, um eine Expression der eingeschleusten DNA in der Ziel- oder Wirtszelle zu ermöglichen.

Ein weiterer Aspekt der Erfindung ist dementsprechend ein Verfahren zur Gewinnung der erfindungsgemäßen Enzyme und Proteine unter Einsatz eines oder mehrerer der erfindungsgemäßen transformierten Mikroorganismen. Das Verfahren umfaßt, mindestens einen durch erfindungsgemäße DNA, insbesondere eine der im Sequenzprotokoll angegebenen cDNAs, unter Kontrolle eines aktiven Promotors transformierten Mikroorganismus, wie vorstehend definiert, zu züchten und das erfindungsgemäße Enzym aus den Mikroorganismen und gegebenenfalls auch dem Kulturmedium zu isolieren. Das Verfahren erstreckt sich selbstverständlich auch auf die Gewinnung von den erfindungsgemäßen Enzymen aus *Solanum tuberosum, Nicotiana tabacum* und *Arabidopsis thaliana* abgeleiteten Enzymen bzw. Proteinen, wie sie vorstehend unter i) definiert sind.

Verfahren zur Züchtung transformierter Mikroorganismen sind dem Fachmann wohlbekannt. Die Isolierung des exprimierten Enzyms kann beispielsweise gemäß dem in Beispiel 5 beschriebenen Verfahren mittels Metall-Chelat-Chromatographie oder alternativ durch Chromatographie an Säulen, die gegen das Enzym gerichtete Antikörper an das Packungsmaterial gebunden enthalten, erfolgen.

### vi) Transgene Pflanzen

Die Erfindung umfaßt gleichfalls transgene Pflanzen, die mittels einer erfindungsgemäßen DNA-Sequenz bzw. eines entsprechenden Konstrukts transformiert worden sind. Es können so z.B. transgene Pflanzen erhalten werden, bei denen eine GnTI-Defizienz, z.B. aufgrund eines fehlenden oder schadhaften *GntI*-Gens oder aufgrund von Defekten in den regulatorischen Bereichen dieses Gens, durch Komplementierung durch ein von den im Sequenzprotokoll angegebenen cDNA-Sequenzen abgeleitetes Konstrukt, dessen Expression unter Kontrolle eines aktiven konstitutiven oder induzierbaren, *ggf*. zusätzlich gewebespezifischen Promotors steht, beseitigt worden ist. Das aufgrund der in dem Konstrukt enthaltenen erfindungsgemäßen DNA exprimierte GnTI-Enzym oder Protein mit GnTI-Aktivität komplementiert in diesem Falle die in der Ausgangspflanze fehlende GnTI-Aktivität.

Gleichfalls in Betracht gezogen werden transgene Pflanzen, in denen die in der Ausgangspflanze bereits vorhandene GnTI-Aktivität durch zusätzliche Expression des durch ein erfindungsgemäßes Konstrukt eingeschleusten *GntI*-Transgens erhöht ist. Ein bei der Untersuchung des Enzyms N-Acetylglucosaminyltransferase I in Pflanzen bestehendes Hauptproblem war bislang die überaus geringe Expression des *GntI*-Gens *in vivo,* verbunden mit einer überaus geringen Enzymaktivität, die entsprechend schwer nachzuweisen war. Durch Coexpression einer erfindungsgemäßen DNA kann das Problem zu geringer GnTI-Enzymaktivität bei Pflanzen behoben werden.

In diesem Falle kann es bevorzugt sein, für die Transformation von Pflanzen erfindungsgemäße DNA einzusetzen, die zusätzlich einen Sequenzabschnitt umfaßt, der nach Expression eine vereinfachte Detektierung und/oder Anreicherung bzw. Reinigung des Proteinproduktes mit GnTI-Aktivität ermöglicht. Beispielsweise gelingt dies durch Einsatz einer speziellen DNA-Sequenz für die Expression eines rekombinanten GnTI-Enzyms, die eine N- oder C-terminale Sequenzverlängerung trägt, die einen Affinitätsmarker kodiert. Ist zusätzlich ein Aminosäuresequenzabschnitt zwischen GnTI-Enzym und Affinitätsmarker vorgesehen, der eine Erkennungsstelle für eine spezifische Protease darstellt, kann durch nachträglichen Einsatz dieser spezifischen Protease die N- oder C-terminale Sequenzverlängerung von dem GnTI-Enzym abgespalten und das GnTI-Enzym dadurch isoliert erhalten werden.

Ein Beispiel hierfür ist der Einsatz einer erfindungsgemäßen DNA-Sequenz, die das rekombinante GnTI-Enzym mit einer C-terminalen Sequenzverlängerung, die den Affinitätsmarker AWRHPQFGG "(Strep-tag"; Ref. 39) kodiert, und einer dazwischenliegenden Protease-Erkennungsstelle, IEGR, kodiert. Die Expression der erfindungsgemäßen DNA liefert GnTI-Enzyme mit der angegebenen C-terminalen Sequenzverlängerung, über welche die exprimierten Proteinmoleküle spezifisch an eine Streptavidin-derivatisierte Matrix binden und so isoliert werden können. Mittels der die Aminosäuresequenz IEGR spezifisch erkennenden Protease Faktor Xa kann der GnTI-Anteil der Proteinmoleküle dann freigesetzt werden. Alternativ kann das vollständige Protein von der Streptavidin-derivatisierten Matrix mittels Biotin oder Biotinderivaten abgelöst werden.

Ein weiteres Beispiel stellen erfindungsgemäße DNA-Sequenzen dar, die ein Protein kodieren, das zusätzlich zu einem GnTI-Enzym eine Mehrzahl, z.B. 10, N-terminal angefügte Histidin-Reste ("His-tag") umfaßt. Eine Isolierung bzw. Reinigung der exprimierten Proteine kann aufgrund der N-terminalen Histidin-Reste leicht durch Metall-Chelat-Affinitätschromatographie (z.B. Ni-Sepharose) erfolgen (vgl. auch Beispiel 5).

Die Erfindung umfaßt gleichfalls Teile derartiger transgener Pflanzen, entsprechend transformierte Pflanzenzellen, transgene Samen und transgenes Vermehrungsmaterial.

Ein weiterer zentraler Aspekt der Erfindung ist die Verwendung der vorstehend erläuterten Sequenzinformation zur Gewinnung von Pflanzen mit verminderter oder fehlender N-Acetylglucosaminyltransferase I-Aktivität.

Die Möglichkeiten zum Auffinden von Pflanzen mit verminderter oder fehlender N-Acetylglucosaminyltransferase I-Aktivität aufgrund eines Gendefektes oder fehlenden Gens durch Einsatz erfindungsgemäßer Antikörper oder erfindungsgemäßer "Screening"- oder Hybridisierungssonden wurden vorstehend bereits beschrieben.

Zwei weitere Möglichkeiten bestehen in der erfindungsgemäßen Verwendung von "antisense"- bzw. "sense"-DNA-Konstrukten, die von der DNA-Sequenz eines *GntI*-Gens einer Pflanze abgeleitet sind, zur Erzeugung transgener Pflanzen mit verminderter oder fehlender N-Acetylglucosaminyltransferase I-Aktivität mittels "homology-dependent gene silencing" (vgl. Ref. 16,17). Die DNA-Sequenz, auf die zur Erzeugung der Konstrukte als Ausgangssequenz zurückgegriffen wird, kann dabei von der zu transformierenden Ausgangspflanze selbst oder aber auch von einer anderen Pflanzenvarietät oder -art stammen. Insbesondere finden "antisense"- oder "sense"-Konstrukte, wie sie vorstehend unter den Punkten iii) und iv) erläutert wurden, Verwendung. Die eingesetzten Konstrukte umfassen üblicherweise mindestens 50 bis 200 und mehr Basenpaare.

Insbesondere umfassen die hierfür eingesetzten Konstrukte mindestens 50 bis 200 und mehr Basenpaare mit einer Sequenz, die ausgehend
- von den im Sequenzprotokoll angegebenen cDNA-Sequenzen und/oder den entsprechenden *GntI*-Genen und/oder
- von den vorstehend erläuterten erfindungsgemäßen abgeleiteten DNA-Sequenzen und/oder DNA-Fragmenten und/oder
- von DNA-Sequenzen insbesondere aus anderen Varietäten und Pflanzenarten, die N-Acetylglucosaminyltransferase I kodieren und aufgrund einer Hybridisierung mit Hybridisierungs- oder "Screening"-Sonden, wie sie vorstehend unter Punkt iii) und iv) definiert wurden, unter stringenten Bedingungen aufgefunden werden können,
abgeleitet wird.

Die Konstrukte enthalten in der Regel einen starken konstitutiven oder induzierbaren, ggf. zusätzlich gewebespezifischen Promotor, unter dessen Kontrolle die "antisense"- oder "sense"-DNA-Sequenzabschnitte stehen.

Bei der Erzeugung transgener Pflanzen durch Integration von "antisense"-Konstrukt(en) in das Pflanzengenom oder durch virale Infektion von Ausgangspflanzen oder -pflanzenzellen durch "antisense"-Konstrukt(e) enthaltendes Virus für eine extrachromosomale Propagation und Transkription des "antisense"-Konstrukts oder der "antisense"-Konstrukte in infiziertem Pflanzengewebe ist beabsichtigt, auf RNA-Ebene eine Hybridisierung von Transkripten des *GntI*-Gens mit Transkripten des "antisense"-DNA-Abschnitts zu erzielen, die die Translation der *GntI*-mRNA verhindert. Die Folge ist eine transgene Pflanze mit stark verringerten Gehalten an N-Acetylglucosaminyltrans-ferase I und damit einer stark verringerten entsprechenden Enzymaktivität.

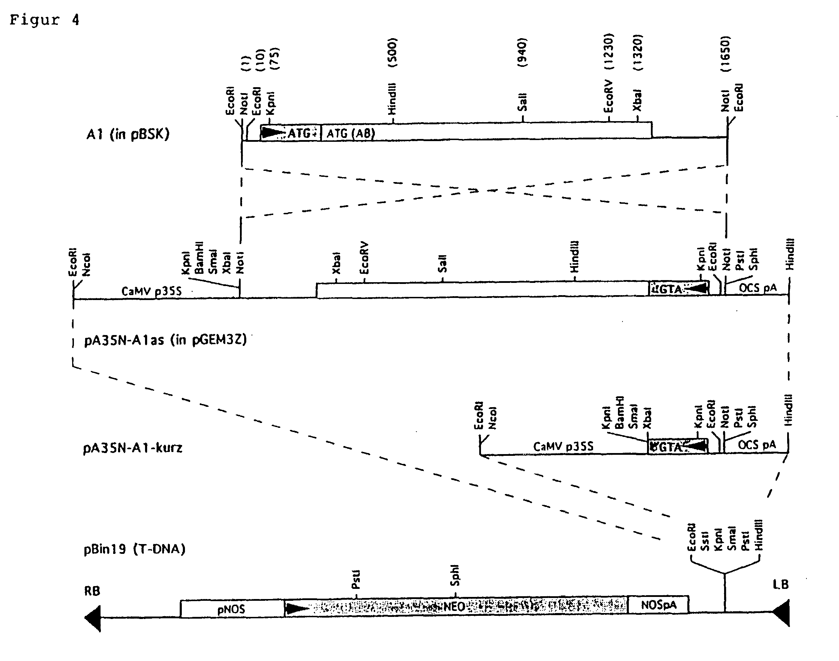

Für die erfindungsgemäße Transformation von Pflanzen mit "antisense"-Konstrukten können beispielsweise Konstrukte eingesetzt werden, die mit einer der kompletten, in Fig. 2 und im Sequenzprotokoll aufgeführten cDNAs oder entsprechenden, in der Regel mindestens 50 bis über 200 Basenpaare umfassenden Abschnitten derselben hybridisieren. Insbesondere bevorzugt ist darüberhinaus die Verwendung von Fragmenten, deren Transkripte zusätzlich zu einer Hybridisierung mit einem Teil des 5'-untranslatierten Bereiches der *GntI*-mRNA führen, an dem oder in dessen Nähe sich normalerweise die Anheftung der Ribosomen vollziehen würde. Beispiele für derartige Konstrukte sind in Fig. 4 gezeigt.

Angesichts des Vorkommens einer Isoform in *Solanum tuberosum* mit wahrscheinlich cytoplasmatischer Lokalisierung aufgrund des fehlenden Membranankers (As 10 bis 29) mit noch unbekannter Funktion kann es wünschenswert sein, lediglich das N-Acetylglucosaminyltransferase I-Enzym zu erfassen, das in den Golgi-Zisternen lokalisiert ist, d.h. nur jenes Enzym, das den Membrananker umfaßt. Ein Grund für diesen Wunsch kann das Bestreben oder im Einzelfalle auch die Notwendigkeit sein, den cytoplasmatischen Metabolismus der Pflanzenzelle, für den die cytoplasmatische N-Acetylglucosaminyltransferase I womöglich von Bedeutung ist, so wenig als möglich zu beeinträchtigen. Zu diesem Zweck können erfindungsgemäß "antisense"-Konstrukte eingesetzt werden, die bzw. deren Transkripte mit einem DNA- oder RNA-Abschnitt des *GntI*-Gens oder der *GntI*-mRNA hybridisieren, der einen Teil des 5'-untranslatierten Bereichs und den kodierenden Bereich - einschließlich des Membranankers - umfaßt. Eine Erst-recküng des Hybridisierungsbereiches bis Position 266 der cDNA in Fig. 2 und SEQ ID NO: 1 wird in der Regel für den genannten Zweck als unschädlich erachtet.

Bei der Erzeugung transgener Pflanzen durch Integration von "sense"-Konstrukten in das Pflanzengenom oder durch virale Infektion von Ausgangspflanzen oder -pflanzenzellen durch "sense"-Konstrukt(e) enthaltendes Virus für eine extrachromosomale Propagation und Expression des oder der Konstrukte in infiziertem Pflanzengewebe wird in Anlehnung an die Arbeiten von Faske et al. (Ref. 17) in Tabak von Hybridisierungsphänomenen dieser Konstrukte mit dem endogenen *GntI*-Gen auf posttranskriptionaler bzw. auf DNA-Ebene ausgegangen, die letzlich die Translation des *GntI*-Gens beeinträchtigen oder verhindern. Das Resultat sind auch in diesem Falle transgene Pflanzen mit verminderter oder sogar fehlender N-Acetylglucosaminyltransferase I-Aktivität.

Verfahren zur stabilen Integration derartiger "antisense"- und "sense"-Konstrukte in das Genom von Pflanzen bzw. zur viralen Infektion von Pflanzen bzw. Pflanzenzellen für eine extrachromosomale Propagation und Transkription/Expression derartiger Konstrukte in infiziertem Pflanzengewebe sind dem Fachmann bekannt. Dazu gehören sowohl der direkte DNA-Transfer (z.B. in Protoplasten mittels Elektroporation oder durch Zusatz eines hochmolekularen Osmotikums sowie biolistische Methoden, bei denen DNA-umhüllte Teilchen in Pflanzengewebe geschossen werden) wie die Verwendung natürlicher Wirt/Vektor-Systeme (z.B. Agrobakterien oder Pflanzenviren). Für eine virale Infektion von Ausgangspflanzen oder -pflanzenzellen durch entsprechende Konstrukte enthaltende Viren für eine extrachromosomale Propagation und Transkription/Expression der Konstrukte in infiziertem Pflanzengewebe stehen eine Reihe spezieller Viren, wie Tabakmosaikvirus (TMV) oder Kartoffelvirus X (potato virus X), zur Verfügung.

Beispielhafte Pflanzen, die für eine derartige Integration in Frage kommen, umfassen dikotyledone wie monokotyledone Kulturpflanzen, insbesondere Solanaceen wie Kartoffel, Tabak, Tomate und Paprika. Zusätzlich wären Banane, Luzerne, Raps, Rüben, Soja, Salat, Mais, Reis und Getreide geeignete Zielpflanzen für den Einsatz homologer "antisense"-Konstrukte. Beispielsweise erscheint die im Sequenzprotokoll angegebene Sequenz aus *Arabidopsis thaliana* insbesondere als Ausgangssequenz für die erfindungsgemäße Transformation von Brassicaceen, wie z.B. Rapspflanzen, mittels "sense"- oder "antisense"-Konstrukten geeignet. Weitere Pflanzen von Interesse sind jegliche Pflanzen, die für Medizin und Forschung interessante Glykoproteine exprimieren.

Allgemein soll festgehalten werden, daß die erfindungsgemäße Transformation von Pflanzen, die in dem entsprechenden Bereich des *GntI*-Gens eine Homologie von ≥70% auf Nukleotidebene zu den eingesetzten erfindungsgemäßen "antisense"- oder "sense"-Konstrukten aufweisen, in der Regel zu erfindungsgemäßen transgenen Pflanzen führt, die die gewünschte Verringerung der N-Acetylglucosaminyltransferase I-Aktivität aufweisen.

Eine weitere Möglichkeit wird ferner in einer zielgerichteten Zerstörung "(Knock out") des N-Acetylglucosaminyltransferase I-Gens durch "gene targeting" mittels homologer Rekombination (Ref. 24) in einer Zielpflanze durch Einsatz eines geeigneten, von der erfindungsgemäßen cDNA-Sequenz abgeleiteten DNA-Fragments gesehen, ähnlich der Vorgehensweise, wie sie beispielsweise für Hefe-Systeme und Säuger etabliert worden ist.

Die Erfindung umfaßt ferner transgene Pflanzen, die mit den vorstehend angesprochenen "antisense"- oder "sense"-Konstrukten bzw. mit diese enthaltenden Viren transformiert worden sind, sowie Teile derartiger transgener Pflanzen, entsprechend transformierte Pflanzenzellen, transgene Samen und transgenes Vermehrungsmaterial.

Verfahren zur Erzeugung transgener Pflanzen, z.B. durch Agrobakterien- oder Virus-vermittelten, wie auch direkten DNA-Transfer, sind dem Fachmann geläufig. Hinsichtlich beispielhafter Pflanzen für eine derartige Transformation gilt das vorstehend ausgeführte.

Die erfindungsgemäßen bzw. erfindungsgemäß gewonnenen Pflanzen mit verminderter oder fehlender N-Acetylglucosaminyltransferase 1-Aktivität können erfindungsgemäß zur Herstellung von Glykoproteinen mit minimalen und einheitlichen, also definierten Zuckerresten eingesetzt werden. Wie bereits erläutert, sind derartige Glykoproteine für Medizin und Forschung von großer Bedeutung. Als preiswerte Rohstoff- und Nahrungsquelle sowie durch ihre problemlose Entsorgung über Kompostierung stellen Pflanzen per se ideale Bioreaktoren dar. Gemäß der vorstehend erläuterten Erfindung können jetzt biotechnologisch oder pharmazeutisch relevante Glykoproteine (z.B. Therapeutika mit niedrigem Antigenpotential für Säuger) in Kulturpflanzen exprimiert werden, in denen GnTI-Aktivität stark gedrosselt ist oder vollständig fehlt.

Die Erfindung umfaßt dementsprechend auch ein Verfahren zur Erzeugung von Glykoproteinen mit minimalen, einheitlichen und definierten Zuckerresten, umfassend das Züchten einer erfindungsgemäßen transgenen Pflanze, von Teilen derartiger Pflanzen oder von erfindungsgemäßen transformierten Pflanzenzellen, die jeweils das gewünschte Glykoprotein exprimieren, und das Isolieren des gewünschten Glykoproteins aus dem gezüchteten Material.

Beispielhafte Kulturpflanzen sind in diesem Zusammenhang Solanaceen, insbesonders Kartoffel, Tabak, Tomate, und Paprika. Des weiteren kommen Banane, Luzerne, Raps, Rüben, Soja, Salat, Mais, Reis und Getreide in Frage.

Die Sequenz der enzymatisch gesteuerten, pflanzenspezifischen N-Glykan-Modifikationen, denen sekretorische Glykoproteine bei Passage durch den Golgi-Apparat höherer Pflanzen unterliegen, ist in Fig. 1 schematisch dargestellt. Die Blockierung der Biosynthese durch fehlende oder unzureichende N-Acetylglucosaminyltransferase I-(GlcNac-Transferase I)-Aktivität in einer Pflanze führt dazu, daß anstelle "komplexer" Glykane hauptsächlich Glykane des Man₅GlcNAc₂-Typs, also Glykoproteine mit einheitlichen und wohldefinierten Zuckerresten gebildet werden, die für Medizin und Forschung von überaus hoher Bedeutung sind.

Hierzu können die Gene für die gewünschten Glykoproteine in ihren natürlichen Erzeugerpflanzen exprimiert werden, die erfindungsgemäß z.B. mittels "antisense"- oder "sense"-Konstrukten zu transgenen Pflanzen mit verminderter oder fehlender N-Acetylglucosaminyltransferase 1-Aktivität transformiert wurden.

Es besteht jedoch auch die Möglichkeit, erfindungsgemäße transgene Pflanzen mit verminderter oder fehlender N-Acetylglucosaminyltransferase I-Aktivität einzusetzen, die zusätzlich mit dem Gen für das gesuchte Glykoprotein transformiert worden sind. Hierzu können Konstrukte eingesetzt werden, die das Gen für das gesuchte Glykoprotein unter der Kontrolle eines starken, konstitutiven oder induzierbaren, ggf. zusätzlich gewebespezifischen Promotors enthalten und die zu einer Integration des Gens in das Genom der Pflanze führen. Alternativ kann die Transformation auch durch virale Infektion durch ein das Gen für das gesuchte Glykoprotein enthaltendes Virus für eine extrachromosomale Propagation und Expression des Gens erfolgen. Das Glykoprotein kann dann in der jeweiligen Wirtspflanze exprimiert und daraus gewonnen werden.

Es kann dabei selbstverständlich alternativ auch so vorgegangen werden, daß zunächst eine Transformation mit einem Expressionskonstrukt oder Virus, das die kodierende DNA des Glykoproteins enthält, vorgenommen wird und erst danach eine weitere Transformation mit einem oder mehreren erfindungsgemäßen "antisense"- oder "sense"-Konstrukten oder einem oder mehreren Viren, die entsprechende DNA enthalten, erfolgt. Eine gleichzeitige Transformation mit beiden Konstrukten oder mit einem Virus, das sowohl das "antisense"- oder "sense"-Konstrukt als auch das das gesuchte Glykoprotein kodierende Gen enthält, ist ebenfalls möglich ("Huckepack"-Version).

Im Rahmen der Erfindung wird auch eine virale Überinfektion erfindungsgemäßer transgener Pflanzen, bei denen bereits eine Integration des "antisense"/"sense"-Konstrukts und/oder des das gesuchte Glykoprotein kodierenden Gens im Genom vorliegt, durch Viren, die "antisense"/"sense"-Konstrukt und/oder das das gesuchte Glykoprotein kodierende Gen enthalten, für eine zusätzliche extrachromosomale Propagation und Transkription bzw. Expression dieser DNA in Betracht gezogen. Hierdurch können die Konzentrationen an "antisense"- bzw. "sense"-DNA oder exprimiertem Glykoprotein in den transgenen Pflanzenzellen erhöht werden.

Für die erfindungsgemäße Gewinnung definiert glykosylierter Glykoproteine kann sich eine Verwendung gewebespezifischer Promotoren beispielsweise in Fällen als sinnvoll erweisen, wenn beabsichtigt ist, die gewünschten Glykoproteine nur aus bestimmten Pflanzenteilen, wie der Knolle oder den Wurzeln, zu gewinnen. Für eine ganze Reihe von Pflanzengeweben stehen heute gewebespezifische Promotoren zur Verfügung, die eine Expression von Fremdgenen speziell nur in diesen Geweben bewirken. Beispielhaft können hier knollenspezifische Promotoren, wie Patatin Klasse I- (Ref. 26) und Proteinase Inhibitor II-Promotoren (Ref. 27) aufgeführt werden. Beide Promotoren zeigen unter bestimmten Bedingungen ebenfalls Expression in Blattgewebe, d.h. können durch hohe Metabolitgehalte (wie z.B. Saccharose) und im Fall des Proteinase Inhibitor II-Promotors auch durch mechanische Verwundung oder Besprühen mit Abscisin- bzw. Jasmonsäure induziert werden.

Eine Verwendung gewebespezifischer Promotoren kann auch dann angezeigt sein, wenn sich die für die Transformation eingesetzte erfindungsgemäße DNA-Sequenz bzw. deren Transkriptions- oder Translationsprodukte für bestimmte Pflanzenteile als abträglich erweisen, z.B. durch negative Einwirkung auf den Metabolismus der entsprechenden Pflanzenzellen.

Als beispielhaftes Ziel-Glykoprotein kommt humane Glucocerebrosidase zur Therapie der erblichen "Gaucher"-Krankheit (Ref. 25) in Frage. Zur Gewinnung von humaner Glucocerebrosidase (GC) mit einheitlichen und definierten Zuckerresten können beispielsweise erfindungsgemäße mittels "antisense"-DNA transformierte Pflanzen mit dem Gen für humane Glucocerebrosidase transformiert werden. Dafür wird die cDNA-Sequenz für humane Glucocerebrosidase (Ref. 38) mittels PCR unter Verwendung genspezifischer Primer am 3'-Ende so modifiziert, daß das rekombinante Enzym eine C-terminale Sequenzverlängerung trägt, die einen Affinitätsmarker (z.B. AWRHPQFGG, "Strep-tag"; Ref. 39) und gegebenenfalls auch eine Protease-Erkennungsstelle (z.B. IEGR) zwischen GnTI-Enzymabschnitt und Affinitätsmarker kodiert. Die so veränderte GC-cDNA-Sequenz wird unter Verwendung eines starken und *ggf*. gewebespezifischen Promotors (z.B. für Kartoffel unter Kontrolle des knollenspezifischen B33-Patatin-Promotors) in erfindungsgemäßen *GntI*-antisense-Pflanzen exprimiert, so daß das in diesen Pflanzen synthetisierte Enzym ausschließlich wohldefinierte N-Glykane trägt. Der Affinitätsmarker soll die Anreicherung des rekombinanten Enzyms aus den transgenen Pflanzen erleichtern. In diesem Falle binden die exprimierten Proteinmoleküle ("GC-Strep"-Moleküle) über die Affinitätsmarkersequenz an eine Streptavidin-derivatisierte Matrix und können von dieser mittels Biotin oder Biotinderivaten abgelöst werden. Die Ablösung von der Streptavidin-derivatisierten Matrix kann auch mittels katalytischer Mengen einer Protease erfolgen, die eine Spezifität für die zwischen dem GnTI-Enzymabschnitt und dem Affinitätsmarker befindliche Protease-Erkennungsstelle aufweist. In diesem Falle wird nur der GnTI-Enzymabschnitt von der Matrix abgelöst. Dies kann insbesondere in dem Falle von Vorteil sein, wenn die Affinitätsmarkersequenz eine nachteilige Wirkung auf die GnTI-Aktivität ausübt.

Die Man₅GlcNAc₂-Glykane der aus den erfindungsgemäßen Pflanzen gewonnenen Glucocerebrosidase werden aufgrund ihrer terminalen Mannose-Reste von Makrophagen als Aufnahmesignal erkannt und können daher direkt zur Therapie der erblichen "Gaucher-Krankheit" eingesetzt werden. Eine Therapie ist derzeit nur durch aufwendige Isolierung und Deglykosylierung nativer Glucocerebrosidase möglich (Ref. 25).

Die Herstellung rekombinanter Glykoproteine läßt sich dementsprechend durch den Einsatz pflanzlicher *GntI*-Sequenzen im Vergleich zu herkömmlichen Verfahren, z.B. der technisch aufwendigen chemischen Deglykosylierung gereinigter Glykoproteine (Ref. 25) oder einer schwierigen und teuren Produktion in GnTIdefizienten tierischen Zellinien (Ref. 7,10), stark vereinfachen.

Erläuterung der Figuren:
- Fig. 1:: Sequenz der pflanzenspezifischen N-Glykan-Modifikationen, denen sekretorische Glykoproteine bei Passage durch den Golgi-Apparat höherer Pflanzen unterliegen (Ref. 28). Der Biosyntheseblock zu "komplex"-modifizierten Glykanen beruht auf einem Defizit an GnTI-Aktivität (hervorgerufen entweder durch defektes oder fehlendes GnTI-Enzym oder durch effektive Drosselung der *GntI*-Genexpression) und ist durch ein Kreuz markiert. Bedeutung der Symbole: (F) Fucose-Reste, (X) Xylose-Reste, (●) GlcNac-Reste, (□) Mannose-Reste.
- Fig. 2:: Vollständige cDNA-Sequenz einer pflanzlichen GnTI aus Kartoffel (*Solanum tuberosum* L.) und davon abgeleitete Aminosäuresequenz. Exemplarisch ist die vollständige cDNA der *GntI*-Isoform mit Membrananker aus Kartoffel-blattgewebe (A1) dargestellt. Die EcoRI/NotI-Linker an den 5'- und 3'-Enden der cDNA sind durch Fettdruck hervorgehoben, die Bindestellen der für die RT-PCR-Sonde verwendeten degenerierten Oligonukleotide sind unterstrichen. Im Gegensatz zu bereits publizierten tierischen GnTI-Sequenzen enthält die abgeleitete Proteinsequenz der Kartoffel cDNA-Klone eine potentielle N-Glykosylierungsstelle: Asn-X(ohne Pro)-Ser/Thr, die mit einem Stern markiert ist. Die Region des Membranankers ist kursiv hervorgehoben (As 10 bis 29). Der Beginn der möglicherweise im Cytosol lokalisierten Isoform (A8) ist durch einen Pfeil gekennzeichnet.
- Fig. 3:: A, Identitäts- bzw. Ähnlichkeitsgrad der abgeleiteten Aminosäuresequenz einer vollständigen *GntI*-cDNA-Sequenz aus Kartoffel (A1) im Vergleich mit anderen, aus Datenbanken ausgewählten GnTI-Sequenzen tierischer Organismen. Identische Aminosäurepositionen (in %) sind fett gedruckt, ähnliche Aminosäurepositionen stehen in Klammern darunter. Bedeutung der Kürzel: Hu, Mensch; Ra, Ratte; Mo, Maus; Ce, *Caenorhabditis elegans* (Spulwurm); St, *Solanum tuberosum* (Kartoffel).
B, Vergleich der abgeleiteten Aminosäuresequenzen verschiedener pflanzlicher *GntI*-cDNA-Klone*.* A_Stb-A1, GnTI aus Kartoffelblatt; B_Ntb-A9, GnTI aus Tabakblatt (A9); C_Atb-Full, GnTI aus *Arabidopsis thaliana*. Identische As sind schwarz, ähnliche As hellgrau markiert.
- Fig. 4:: Klonierungsschema der verwendeten *GntI*-"antisense"-Konstrukte. In die SalI-Schnittstelle der Polylinkerregion des pflanzlichen Expressionsvektors pA35 (Ref. 29) wurde nach Auffüllen der Enden ein NotI-Linker eingeführt (= pA35N) und die vollständige Al-*GntI*-cDNA über NotI in pA35N inseriert. Das entsprechende "antisense"-Konstrukt (= pA35N-Alas) wurde über EcoRI und HindIII in den binären Vektor pBin19 (Ref. 30) inseriert. Des weiteren wurde nach PCR-Amplifikation ein ca. 270 Bp umfassendes 5'-Fragment der A1-*GntI*-cDNA über XbaI- und NotI-Schnittstellen in pA35N in "antisense"-Orientierung kloniert (= pA35N-A1-kurz) und ebenfalls in pBin19 inseriert. Abkürzungen: Zahlen in Klammern, Positionsangaben der Restriktionsschnittstellen in der A1-*GntI*-cDNA (in Basenpaaren); pBSK, Klonierungsvektor (Stratagene); pGEM3Z, Klonierungsvektor (Promega); CaMV p35S, konstitutiver Blumenkohl-Mosaikvirus-35S-Promotor; OCSpA, Octopinsynthase-Polyadenylierungssignal; pNOS, Nopalinsynthase-Promotor; NEO, Neomycinphosphotransferase (Selektionsmarker, vermittelt Kanamycinresistenz); NOSpA, Nopalinsynthase-Polyadenylierungssignal; LB/RB, "left/right border" der T-DNA des binären Vektors; Pfeil, Translationsstart (ATG); A8, Beginn der potentiell cytosolisch lokalisierten *GntI*-Isoform (7 As-Austausche im Vergleich zu A1).

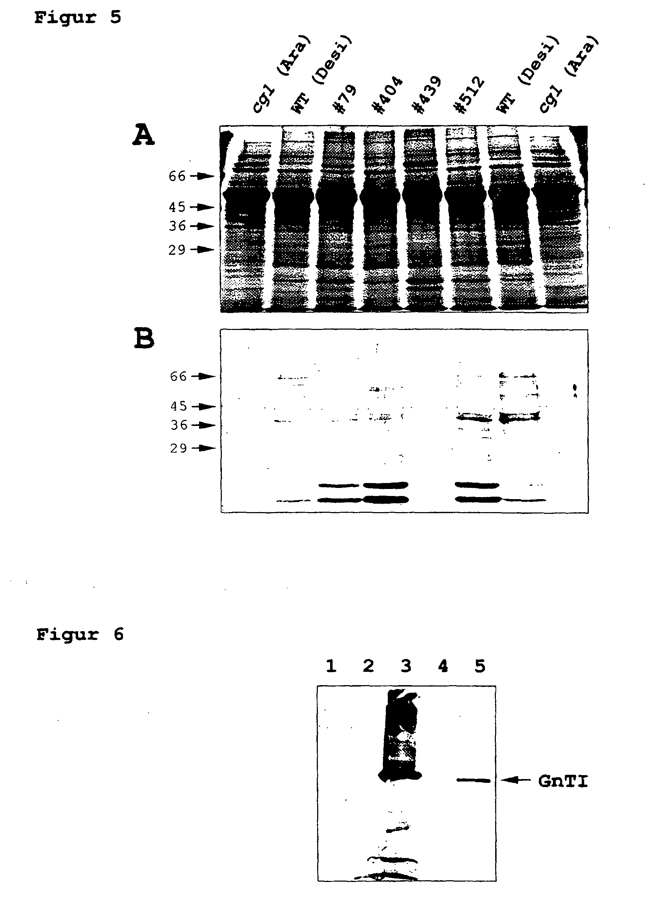

- Fig. 5:: Ausmaß der Suppression komplexer Glykoprotein-Modifikation in transgenen Kartoffelpflanzen, die mit dem langen *GntI*-"antisense"-Konstrukt (vgl. Fig. 4) transformiert wurden. A, Coomassie-gefärbtes SDS-Gel von Blattextrakten; B, "Western-Blot"-Analyse (Ref. 13,33) von Parallelproben mit einem "complex-glycan"-Antiserum (Ref. 12,13). Die Spuren enthalten je 30 µg Gesamtprotein: *cgl*(Ara), Arabidopsis *cgl*-Mutante (Ref. 13); WT(Desi), Kartoffel Wildtyp; die Nummern bezeichnen einzelne transgene Kartoffelpflanzen, die Pfeile Molmassenstandards von 66, 45, 36 und 29 kD.
- Fig. 6:: Nachweis der Spezifität des erzeugten GnTI-Antiserums nach Zellfraktionierung (Ref. 40) von Tabak-Kallusmaterial. Für die "Western Blot"-Analyse (Ref. 13, 33) wurden pro Spur je 30 µg Protein aufgetragen. Das Antiserum wurde in einer Verdünnung von 1:1000 eingesetzt. Spur 1, Homogenat nach Abtrennung von Zelltrümmern; Spur 2, Vesikel-Fraktion nach Säulenchromatographie; Spur 3, Saccharose-Gradient-Fraktion I (Mikrosomen); Spur 4, Saccharose-Gradient-Fraktion II (Plastiden); Spur 5, für die Immunisierung verwendetes Antigen (rekombinantes GnTI-Fusionsprotein; Pfeil, Molmasse von ca. 49 kD.

### Erläuterung der im Text verwendeten Abkürzungen:

As, Aminosäure(n); Bp, Basenpaar(e); EMS, Ethylmethansulfonat (mutagene Chemikalie); F2, zweite Filialgeneration; Fuc, Fucose; Glc, Glucose; GlcNac, N-Acetylglucosamin; GnTI, N-Acetylglucosaminyltransferase I (EC 2.4.1.101); *GntI,* Gen für GnTI (Kern-codiert); kD, Kilodalton; Man, Mannose; PCR, Polymerasekettenreaktion; PAGE, Polyacrylamidgelelektrophorese; Ref., Referenz; RT-PCR, Reverse Transkription gekoppelt mit Polymerasekettenreaktion; SDS, Natriumdodecylsulfat; Var., Varietät; Xyl, Xylose.

Die Erfindung wird nun anhand von Beispielen eingehender erläutert. Die Beispiele werden lediglich zur Veranschaulichung der Erfindung aufgeführt und beschränken die Erfindung in keiner Weise.

### Bsp. 1: Isolierung und Charakterisierung von pflanzlichen GntI-cDNA-Klonen.

Aus Kartoffel- und Tabak-Blattgewebe wurde Gesamt-RNA isoliert und mittels RT-PCR in Kombination mit degenerierten Primern (Vorgehen analog zu Ref. 31), die von konservierten Aminosäurebereichen bekannter GnTI-Sequenzen aus tierischen Organismen abgeleitet wurden ("sense"-Primer 1*, 5'-TG(CT) G(CT)I (AT) (GC) I GCI TGG (AC)A(CT) GA(CT) AA(CT)-3'; "antisense"-Primer 3*, 5' -CCA ICC IT(AG) ICC (ACGT)G(CG) (AG)AA (AG)AA (AG) TC-3' ; je 30 pMol Primer pro 50 µl PCR-Ansatz bei 55°C "annealing"-Temperatur und 45 Zyklen), cDNA-Fragmente von ca. 90 Bp amplifiziert. Nach Gelelution wurden die Enden der PCR-Produkte repariert (d.h. durch DNA-Polymerase I glatte Enden erzeugt und mit T4-Polynukleotid-Kinase phosphoryliert) und in die EcoRV-Schnittstelle von pBSK (Stratagene) kloniert. Die Identität der abgeleiteten Aminosäuresequenzen der Kartoffel und Tabak RT-PCR-Produkte konnte durch Vergleich mit bekannten GnTI-Sequenzen zwischen den Primern (Pfeile) als homolog bestätigt werden; ⇒Q(R/M)QFVQDP(D/Y)ALYRS⇐ (homologe As unterstrichen). Von je einem der Klone wurden mittels PCR radioaktiv markierte Sonden synthetisiert (Standard-PCR-Ansatz mit degenerierten Primern wie oben, Nukleotidmischung ohne dCTP, dafür mit 50 µCi α-³²P-dCTP [>3000 Ci/mMol] versetzt) und verschiedene cDNA-Banken mit den entsprechenden homologen Kartoffel- bzw. Tabak-Sonden auf *GntI*haltige Klone durchgemustert (Vorgehen analog zu Ref. 31; "stringente" Hybridisierungsbedingungen wurden bereits oben im Text definiert). Die cDNA-Banken wurden mit mRNA aus jungen, noch wachsenden Pflanzenteilen ("sink"-Gewebe) hergestellt. Nach cDNA-Synthese und Ligieren von EcoRI/NotI-Adaptoren (cDNA-Synthese-Kit, Pharmacia) wurde mit EcoRI-kompatiblen Lambda-Armen ligiert, diese verpackt und damit *E*. *coli* XLI-Blue-Zellen transfiziert (Lambda ZAPII Klonierungs- und Verpackungssystem, Stratagene). Nach Amplifikation der Banken wurde je ein vollständiger *GntI*-Klon aus einer Kartoffelblatt-"sink"-Bank (Al gemäß Fig. 2 und SEQ ID NO: 1) und einer Tabakblatt-"sink"-Bank (A9 gemäß SEQ ID NO: 3), sowie zwei weitere Klone aus einer Knollen-"sink"-Bank isoliert (A6,A8). Die abgeleiteten GnTI-Aminosäuresequenzen enthalten im Gegensatz zu denen von Tieren eine potentielle N-Glykosylierungsstelle, Asn-X(ohne Pro)-Ser/Thr. Eine der *GntI*-cDNA-Sequenzen aus Knollen trägt vor dem ersten Methionin Stop-Codons in allen drei Leserahmen (A8). Der codierende Bereich ist zu dem längeren Knollenklon (A6) stark homolog (nur 2 As-Austausche), trägt jedoch eine völlig andere 5'-untranslatierte Region. Des weiteren fehlt der für das Golgi-Enzym charakteristische Membrananker, so daß diese *GntI*-Isoform im Cytosol lokalisiert sein könnte.Sequenzvergleiche wurden mithilfe der "gap"- bzw. "pileup"- und "box"-Option des GCG-"software"-Pakets (J Devereux, P Haeberli, O Smithies (1984) Nucl Acids Res 12: 387-395) erstellt und zeigen, daß die abgeleiteten pflanzlichen GnTI-Aminosäuresequenzen zu denen aus tierischen Organismen nur 30-40% Identität und 57-59% Ähnlichkeit aufweisen (Fig. 3A), untereinander aber hoch hamolog sind (75-90% Identität, Fig. 3B) .

Bei *Arabidopsis thaliana* wurde analog vorgegangen, wobei zur Herstellung einer spezifischen Sonde zunächst eine GnTI-Teilsequenz durch RT-PCR mit *GntI* "sense"-Primer 4A (5'-ATCGGAAAGCTTGGATCC CCA GTG GC(AG) GCT GTA GTT GTT ATG GCT TGC-3'; HindIII-Schnittstelle unterstrichen, BamHI fett gedruckt) und "antisense"-Primer 3*, wie vorstehend definiert, amplifiziert wurde. Aus einer Phagenbank (Lambda Uni-ZAP) wurde mit dieser Sonde zunächst ein 5'-unvollständiger cDNA-Klon isoliert. Durch Vektor-Insert-PCR wurde das fehlende 5'-Ende aus einer weiteren Bank amplifiziert und über eine unique SpeI-Schnittstelle im 5'-Bereich zu einer vollständigen cDNA-Sequenz zusammengesetzt. Die durch Sequenzierung ermittelte Nukleinsäuresequenz ist in SEQ ID NO: 5 aufgeführt.

### Bsp. 2: Funktionelle Komplementierung eines GnTI-Defekts mit GntI-cDNA bei transienter Expression in Protoplasten der Arabidopsis thaliana cgl-Mutante.

Ca. 4 Wochen nach Aussaat wurden Protoplasten aus Blättern steril angezogener *cgl*-Mutanten ("Nichtfärber"-Pflanzen nach 5 Rückkreuzungen, Ref. 13) isoliert und mit Expressionskonstrukten der vollständigen *GntI-*cDNA-Sequenzen (NotI-cDNA-Fragmente, vgl. Fig. 4) in "sense"- (pA35N-Als bzw. pA35N-A9s) oder "antisense"-Orientierung (pA35N-Alas bzw. pA35N-A9as) transformiert und für 96 Std. bei Raumtemperatur abgedunkelt kultiviert (je 50 µg Plasmid-DNA pro 1 Mio. Protoplasten, PEG-Methode nach Ref. 32). Anschließende SDS-PAGE der Protoplastenextrakte und "Western Blot"-Analyse (analog zu Ref. 13,33) zeigte funktionelle Komplementierung des GnTI-Defekts, d.h. "komplexe" Glykosylierung zahlreicher Proteinbanden bei transienter Expression der Kartoffel A1- und Tabak A9-"sense"-, nicht aber der entsprechenden "antisense"-Konstrukte in Protoplasten der Arabidopsis *cgl*-Mutante (Daten nicht gezeigt).

### Bsp. 3: Klonierung der binären Expressionskonstrukte pBin-35-Alas und pBin-35-A1-kurz (vgl. Fig. 4).

In die SalI-Schnittstelle der Polylinkerregion (entspricht pUC18) des pflanzlichen Expressionsvektors pA35 (Ref. 29) wurde nach Auffüllen der Enden ein NotI-Linker eingeführt (pA35N), und die vollständige A1-*GntI*-cDNA (Nukleotide 9 bis 1657, gemäß der cDNA in Fig. 2) über NotI in pA35N inseriert ("sense"-Konstrukt pA35N-Als bzw. "antisense"-Konstrukt pA35N-Alas). Die Expressionskassetten der "sense" bzw. "antisense"-Konstrukte wurden über die terminalen Schnittstellen (NcoI-Schnittstelle aufgefüllt, mit HindIII partial nachverdaut) als ca. 2410 Bp-Fragment isoliert und in die EcoRI- (aufgefüllt) und HindIII-Schnittstellen des binären Vektors pBin19 (Ref. 30) inseriert (= pBin-35-Als bzw. pBin-35-Alas). Durch Fusion mit der ebenfalls aufgefüllten NcoI-Schnittstelle des Fragments wird die EcoRI-Schnittstelle des Vektors regeneriert. Zusätzlich wurde in einem Standard-PCR-Ansatz ("sense"-Primer: KS-Sequenzprimer (Stratagene), verlängert für PCR, 5'-GGC CCC CCC TCG AGG TCG ACG GTA TCG-3'; "antisense"-Primer: 5'-GGGCCTCTAGA*CTCGAG* AGC (CT)AC TAC TCT TCC TTG CTG CTG GCT AAT CTT G-3', XbaI-Schnittstelle unterstrichen, XhoI-Schnittstelle kursiv) bei 50°C "annealing"-Temperatur ein 5'-Fragment der GntI-cDNA amplifiziert (Nukleotide 9 bis 261, gemäß der cDNA in Fig. 2 und SEQ ID NO: 1). Das PCR-Produkt wurde mit XbaI (im "antisense"-Primer) und NotI (im 5'-Linker der cDNA) verdaut, als ca. 260 Bp-Fragment isoliert und in pA35N kloniert (= pA35N-A1-kurz). Die Expressionskassette des kurzen "antisense"-Konstrukts wurde als EcoRI/HindIII-Fragment (ca. 1020 Bp) ebenfalls in pBin19 inseriert (= pBin-35-A1-kurz).

### Bsp. 4: Transformation von Agrobakterien durch die binären GntI-Konstrukte und Regeneration von transgenen Kartoffel- bzw. Tabakpflanzen aus infizierten Blattscheiben.

Die binären "antisense"-*GntI*-Konstrukte (pBin-35-Alas bzw. pBin-35-A1-kurz) wurden in den Agrobakterien-Stamm GV2260 transformiert (Ref. 34,35). Mit den rekombinanten Agrobakterienlinien wurden exemplarisch sterile Blattscheiben von Kartoffelpflanzen der Var. Désirée bzw. Tabakpflanzen der Var. Wisconsin 38 infiziert (50 µl einer frischen Übernachtkultur in 10 ml flüssigem 2MS-Medium: 2% Saccharose in Murashige & Skoog Salz/Vitamin-Standard-Medium, pH 5,6; Blattstückchen ohne Mittelrippen; Cokultivierung 2 Tage dunkel in Pflanzen-Klimakammern). Nach Waschen der infizierten Blattstückchen in 2MS-Medium mit 250 µg/ml Claforan wurden aus diesen in Gewebekultur unter Kanamycin-Selektion transgene Pflanzen regeneriert (Kartoffelprotokoll Ref. 26; Tabakprotokoll Ref. 36) und auf reduzierte GnTI-Aktivität getestet (exemplarisch in Fig. 5 für transgene Kartoffelpflanzen gezeigt). Wie aus Fig. 5 ersehen werden kann, war die "antisense"-Suppression komplexer Glykoprotein-Modifikation in der transgenen Kartoffelpflanze #439 erfolgreich. Die festgestellte Drosselung der komplexen Glykoprotein-Modifikation war in dieser Transformante über den gesamten Untersuchungszeitraum von mehreren Monaten stabil und wurde in drei, jeweils im Abstand von ca. 1 Monat durchgeführten Tests bestätigt. Analoge Ergebnisse wurden für die entsprechenden transgenen Tabakpflanzen erhalten.

### Bsp. 5: Gewinnung von rekombinantem Kartoffel-GnTI-Protein (zur Antikörperproduktion).

Mit Hilfe des pET-Systems (Novagen) wurde rekombinante GnTI mit 10 zusätzlichen N-terminalen Histidin-Resten ("His-tag") in *E. coli* erzeugt und durch Metall-Chelat-Affinitätschromatografie gereinigt. Ein cDNA-Fragment, das die Nukleotide 275-1395 der Kartoffel *GntI*-cDNA umfaßt (entsp. As 75-446, Fig. 2 bzw. SEQ ID NO: 1 und 2), wurde mittels Standard-PCR ("annealing"-Temperatur 50°C, 30 Zyklen, Ref. 31) amplifiziert ("sense"-Primer GntI-5'fus: 5'-GATGGATCC CTC GAG AAG CGT CAG GAC CAG GAG TGC CGG C-3'; "antisense"-Primer GntI-3'stop: 5'-ATCCCGGGATCCG CTA CGT ATC TTC AAC TCC AAG TTG-3'; XhoI- bzw. BamHI Schnittstellen unterstrichen, Stop-Codon kursiv), und über die Restriktionsschnittstellen der synthetischen Primer (5'-XhoI-*GntI*-BamHI-3') in den Vektor pET16b (Novagen) inseriert (= pET-His-A1). Nach Vermehrung und Analyse in *E. coli* XLI-Blue (Stratagene) wurde das Konstrukt als Glycerinkultur archiviert. Zur Überexpression wurden kompetente *E. coli* BL21(DE3)pLysS-Zellen (Novagen) mit pET-His-Al transformiert. Zugabe von IPTG (Isopropyl-1-thio-β-D-galaktopyranosid, ad 0,5-2 mM) zu einer logarithmisch wachsenden BL21-Kultur induziert zunächst die Expression von (bakterienchromosomaler) T7-RNA-Polymerase und damit ebenfalls die Expression des rekombinanten Fusionsproteins, das in pET-Vektoren (Novagen) unter T7-Promotor-Kontrolle steht. Aus induzierten BL21:pET-His-Al-Zellen wurde rekombinante Kartoffel-GnTI mit "His-tag" unter denaturierenden Bedingungen (Hersteller-Protokoll, Novagen) mittels Metall-Chelat-Chromatographie an TALON-Matrix (Clontech) gereinigt und die Präparation mittels SDS-PAGE auf Einheitlichkeit überprüft.

### Bsp. 6: Erzeugung von polyklonalen Antikörpern in Kaninchen.

Rekombinante Kartoffel-GnTI (aus Bsp. 5) wurde als Antigen verwendet. Nach Entnahme von einigen Millilitern Prä-Immunserum wurde den Kaninchen in dreiwöchigen Abständen 300-500 µg affinitätsgereinigtes Protein zusammen mit 25 µg GMDP-Adjuvans (Gerbu) subcutan injiziert. Nach drei Basis-Injektionen wurden die Tiere 12 bis 14 Tage nach der jeweiligen Folge-Injektion ("Boost") aus der Ohrvene geblutet, das Serum gewonnen (Ref. 37) und auf Erkennung rekombinanter GnTI in "Western Blot"-Analysen (1:200 bis 1:2000-Verdünnung) getestet. Das Antiserum der "Boosts" mit geringstem Hintergrund-zu-Signal-Verhältnis wurde mit 0,04% Natriumazid versetzt, aliquotiert und bei +4°C bzw, längerfristig bei -20°C gelagert. Wie in Fig. 6 gezeigt, ergaben "Western Blot"-Analysen von Tabak-Kalluszellen (BY-2-Suspensionskultur) ein spezifisches GnTI-Signal in angereicherten Mikrosomenfraktionen, welches anzeigt, daß die gegen das rekombinante Protein hergestellten Antikörper pflanzliche GnTI spezifisch erkennen. Der Nachweis wurde mit angereicherten Mikrosomenfraktionen (ER und Golgi-Vesikel) durchgeführt, da GnTI-Protein, bedingt durch die geringen Mengen, in Pflanzen-Rohextrakten mit der verwendeten "Western-Blot"-Methode nicht nachzuweisen ist.

### Referenzen

1) R Kornfeld, S Kornfeld (1985) Assembly of asparagine-linked oligosaccharides. Annu Rev Biochem 54: 631-664
2) GP Kaushal, T Szumilo, AD Elbein (1988). Structure and biosynthesis of plant N-linked glycans. *In* J Preiss (editor) The Biochemistry of Plants, Vol 14: Carbohydrates. Academic Press, San Diego, CA, pp 421-463
3) L Faye, MJ Chrispeels (1989) Apparent inhibition of β-fructosidase secretion by tunicamycin may be explained by breakdown of the unglycosylated protein during secretion. Plant Physiol 89: 845-851
4) TW Rademacher, RB Parekh, RA Dwek (1988) Glycobiology. Annu Rev Biochem 57: 785-838
5) A Sturm (1991) Heterogeneity of the complex N-linked oligosaccharides at specific glycosylation sites of 2 secreted carrot glycoproteins. Eur J Biochem 199: 169-179
6) K Olden, BA Bernard, MJ Humphries, T Yeo, SL White, SA Newton, HC Bower, JB Parent (1985) Function of glycoprotein glycans. Trends Biochem Sci 10: 78-82
7) P Stanley (1989) Chinese hamster ovary cell mutants with multiple glycosylation defects for production of glycoproteins with minimal carbohydrate heterogeneity. Mol Cell Biol 9: 377-383
8) R Kumar, J Yang, RD Larsen, P Stanley (1990) Cloning and expression of N-acetylglucosaminyltransferase I, the medial Golgi transferase that initiates complex N-linked carbohydrate formation. Proc Natl Acad Sci USA 87: 9948-9952
9) JW Dennis, S Laferte, C Waghorne, ML Breitman, RS Kerbel (1987) β→6 branching of Asn-linked oligosaccharides is directly associated with metastasis. Science 236: 582-585
10) MN Fukuda (1990) HEMPAS disease: genetic defect of glycosylation. Glycobiology 1: 9-15
11) MN Fukuda, KA Masri, A Dell, L Luzzatto, KW Moremen (1990) Incomplete synthesis of N-glycans in congenital dyserythropoetic anemia type II caused by a defect in the gene encoding α-mannosidase II. Proc Natl Acad Sci USA 87: 7443-7447
12) M Laurière, C Laurière, MJ Chrispeels, KD Johnson, A Sturm (1989) Characterization of a xylose-specific antiserum that reacts with the complex asparagine-linked glycans of extracellular and vacuolar glycoproteins. Plant Physiol 90: 1182-1188
13) A von Schaewen, A Sturm, J O'Neill, MJ Chrispeels (1993) Isolation of a mutant *Arabidopsis* plant that lacks N-acetyl glucosaminyl transferase I and is unable to synthesize Golgi-modified complex N-linked glycans. Plant Physiol 102: 1109-1118
14) JK-C Ma, MB Hein (1995) Plant antibodies for immunotherapy. Plant Physiol 109: 341-346
15) AS Moffat (1995) Medical applications: Exploring transgenic plants as a new vaccine source. Science 268: 658-660 (Zusammenfassung von zwei Originalveröffentlichungen in derselben Ausgabe)
16) CB Taylor (1997) Comprehending cosuppression. Plant Cell 9: 1245-1249 (Zusammenfassung von mehreren Originalveröffentlichungen in derselben Ausgabe)
17) M Faske, JE Backhausen, M Sendker, M Singer-Bayrle, R Scheibe, A von Schaewen (1997) Transgenic tobacco plants expressing pea chloroplast *Nmdh* cDNA in sense and antisense orientation: Effects on NADP-MDH level, stability of transformants, and plant growth. Plant Physiol 115: 705-715
18) R Koes, E Souer, A van Houwelingen, L Mur, C Spelt, F Quattrocchio, J Wing, B Oppedijk, S Ahmed, T Maes, T Gerats, P Hoogeveen, M Meesters, D Kloos, JNM Mol (1995) Targeted gene inactivation in petunia by PCR-based selection of transposon insertion mutants. Proc Acad Sci USA 92: 8149-8153
19) EC McKinney, N Ali, A Traut, KA Feldmann, DA Belostotsky, JM McDowell, RB Meagher (1995) Sequence-based identification of T-DNA insertion mutations in *Arabidopsis:* actin mutants *act2-1* and *act4-1*. Plant J 8: 613-622
20) F Altmann, G Kornfeld, T Dalik, E Staudacher, J Glössl (1993) Processing of asparagine-linked oligosaccharides in insect cells. N-acetylglucosaminyl transferase I and II activities in cultured lepidopteran cells. Glycobiology 3: 619-625
21) A Sturm, KD Johnson, T Szumilo, AD Elbein, MJ Chrispeels (1987) Subcellular localization of glycosidases and glycosyltransferases involved in the processing of N-linked oligosaccharides. Plant Physiol 85: 741-745
22) GM Church, W Gilbert (1984) Genomic sequencing. Proc Acad Sci USA 81: 1991-1995
23) J Sambrook, EF Fritsch, T Maniatis (1989) *Molecular cloning: a laboratory manual* (2nd edn), Cold Spring Harbor Laboratory, Cold Spring Harbor, NY
24) H Puchta, B Hohn (1996) From centiMorgans to base pairs: homologous recombination in plants. Plant Sci 1: 340-348
25) NW Barton, FS Furbish, GJ Murray, M Garfield, RO Brady (1990) Therapeutic response to intravenous infusions of glucocerebrosidase in a patient with Gaucher disease. Proc Natl Acad Sci USA 87: 1913-1916
26) M Rocha-Sosa, U Sonnewald, W-B Frommer, M Stratmann, J Schell, L willmitzer (1989) Both developmental and metabolic signals activate the promoter of a class I patatin gene. EMBO J 8: 23-29
27) T Hildmann, M Ebneth, H Pena-Cortes, JJ Sanchez-Serrano, L Willmitzer, S Prat (1992) General roles of abscisic and jasmonic acids in gene activation as a result of mechanical wounding. Plant Cell 4: 1157-1170
28) KD Johnson, MJ Chrispeels (1987) Substrate specificities of N-acetylglucosaminyl-, fucosyl-, and xylosyltransferases that modify glycoproteins in the Golgi apparatus of bean cotyledons. Plant Physiol 84: 1301-1308
29) H Höfte, L Faye, C Dickinson, EM Herman, MJ Chrispeels (1991) The protein-body proteins phytohemagglutinin and tonoplast intrinsic protein are targeted to vacuoles in leaves of transgenic tobacco. Planta 184: 431-437
30) M Bevan (1984) Binary *Agrobacterium* vectors for plant transformation. Nucl Acids Res 12: 8711-8721
31) K Graeve, A von Schaewen, R Scheibe (1994) Purification, characterization and cDNA sequence of glucose-6-phosphate dehydrogenase from potato (*Solanum tuberosum* L.). Plant J 5: 353-361
32) B Damm, R Schmidt, L Willmitzer (1989) Efficient transformation of *Arabidopsis thaliana* using direct gene transfer to protoplasts. *Mol Gen Genet* 213: 15-20
33) A von Schaewen, M Stitt, R Schmidt, L Willmitzer (1990) Expression of a yeast-derived invertase in the cell wall of tobacco and *Arabidopsis* plants leads to accumulation of carbohydrate, inhibition of photosynthesis and strongly influences growth and phenotype of transgenic tobacco plants. EMBO J. 9: 3033-3044
34) R Deblaere, B Bytebier, H De Greve, F Debroeck, J Schell, M van Montagu, J Leemans (1985) Efficient octopine Ti plasmid-derived vectors for *Agrobacterium* mediated gene transfer to plants. Nucl Acids Res 13: 4777-4788
35) R Höfgen, L Willmitzer (1988) Storage of competent cells for Agrobacterium transformation. Nucl Acids Res 16: 9877
36) T Voelker, A Sturm, MJ Chrispeels (1987) Differences in expression between two seed lectin alleles obtained from normal and lectin-deficient beans are maintained in transgenic tobacco. EMBO J 6: 3571-3577
37) E Harlow, D Lane (1988) Antibodies: A Laboratory Manual, Cold Spring Harbor Laboratory Press, Cold Spring Harbor, NY
38) J Sorge, C West, B Westwood, E Beutler (1985) Molecular cloning and nucleotide sequence of human cerebrosidase cDNA. Proc Natl Acad Sci USA 82: 7289-7293
39) TGM Schmidt, A Skerra (1993) The random peptide library-assisted engineering of a C-terminal affinity peptide, useful for the detection and purification of a functional Ig Fv fragment. Prot Engineering 6: 109-122
40) W van der Wilden, NR Gilkes, MJ Chrispeels (1980) The endoplasmic reticulum of mung bean cotyledons: role in the accumulation of hydrolases in protein bodies during seedling growth. Plant Physiol. 66: 390-394

## Patentansprüche

1. Verfahren zur Erzeugung von Glykoproteinen mit minimalen, einheitlichen GlcNAc₂Man₅-Resten, umfassend das Züchten einer transgenen Pflanze, von Teilen transgener Pflanzen oder von transformierten Pflanzenzellen und das Isolieren des gesuchten Glykoproteins aus dem gezüchteten Material **dadurch gekennzeichnet, dass** die transgene Pflanze, die Teile transgener Pflanzen bzw. die transformierten Pflanzenzellen mit einem "antisense"-Konstrukt oder einem "sense"-Konstrukt, umfassend eine "antisense"-DNA bzw. "sense"-DNA bezüglich der DNA-Sequenz eines Gens oder einer cDNA für pflanzliche N-Acetylglucosaminyltransferase I oder eines Teils davon, zur Beseitigung oder Verringerung des N-Acetylglucosaminyltransferase I-Aktivität in diesen transformiert ist bzw. sind, wobei das "antisense"- oder "sense"-Konstrukt gegebenenfalls zusätzlich regulatorische Sequenzen für die Transkription der entsprechenden "antisense"- oder "sense"-DNA enthält.

2. Verfahren nach Anspruch 1, **dadurch gekennzeichnet, dass** für die Transformation ein "antisense"- oder "sense"-Konstrukt bezüglich einer des cDNAs, die N-Acetylglucosaminyltransferase I aus Solanum tuberosum, Nicotiana tabacum oder Arabidopsis thaliana kodieren, verwendet wird.

3. Verfahren nach Anspruch 2, **dadurch gekennzeichnet, dass** für die Transformation ein "antisense"- oder "sense"-Konstrukt eine der in SEQ ID NO: 1, 3 oder 5 angegebenen DNA-Sequenzen verwendet wird.

4. Verfahren nach einem der Ansprüche 1 bis 3, **dadurch gekennzeichnet, dass** die eingesetzte transgene Pflanze zusätzlich mit dem das gesuchte Glykoprotein kodierende Gen transformiert worden ist.

5. DNA, **dadurch gekennzeichnet, dass** sie N-Acetylglucosaminyltransferase I aus Solanum tuberosum kodiert und die in SEQ ID NO: 1 angegebene Nukleotidsequenz umfasst.

6. DNA, **dadurch gekennzeichnet, dass** sie N-Acetylglucosaminyltransferase I aus Nicotiana tabacum kodiert und die in SEQ ID NO: 3 angegebene Nukleotidsequenz umfasst.

7. DNA, die N-Acetylglucosaminyltransferase I aus Arabidopsis thaliana kodiert, **dadurch gekennzeichnet, dass** die DNA die in SEO ID NO 6 angegebene Aminosäuresequenz kodiert oder die in SEQ ID NO: 5 angegebene Nukleotidsequenz umfasst.

8. DNA, **dadurch gekennzeichnet, dass** sie die komplementäre Nukleotidsequenz zu der DNA nach Anspruch 5, 6 oder 7 aufweist.

9. DNA, **dadurch gekennzeichnet, dass** sie durch Substitution, Deletion und/oder Insertion einzelner oder mehrerer Nukleotide und/oder Verkürzung am 5'- und/oder 3'-Ende einer der DNAs nach einem der Ansprüche 5 bis 8 erhalten werden kann mit der Maßgabe, dass die DNA unter stringenten Bedingungen mit der Ausgangs-DNA oder deren komplementärer Sequenz hybridisiert.

10. DNA, **dadurch gekennzeichnet, dass** sie ein Gen darstellt oder Teil eines Gens ist, das das pflanzliche Enzym N-Acetylglucosaminyltransferase 1 kodiert, und
- mit einer der DNA-Sequenzen nach einem der Ansprüche 5 bis 9 und/oder
- mit einer DNA-Sequenz, die von den in den SEQ ID NO: 1, 3 und/oder 5 angegebenen Aminosäuresequenz unter Berücksichtigung des Degeneration des genetischen Codes abgeleitet worden ist,
unter stringenten Bedingungen hybridisiert.

11. DNA-Konstrukt, **dadurch gekennzeichnet, dass** es eine oder mehrere der DNAs gemäß einem der Ansprüche 5 bis 10 umfasst.

12. DNA-Konstrukt, **dadurch gekennzeichnet, dass** es eine "antisense"- oder eine "sense"-DNA bezüglich des DNA-Sequenz nach einem des Ansprüche 5 bis 10 und ggf. regulatorische Sequenzen für die Transkription des "antisense"- bzw. "sense"-DNA umfasst.

13. Vektor, Plasmid, Cosmid, Viren- oder Phagengenom, **dadurch gekennzeichnet, dass** er oder es zumindest eine DNA und/oder ein Konstrukt nach einem der Ansprüche 5 bis 12 umfasst.

14. N-Acetylglucosaminyltransferase I aus Arabidopsis thaliana, **dadurch gekennzeichnet, dass** das Enzym die in SEQ ID NO: 6 angegebene Aminosäuresequenz umfasst.

15. N-Acetylglucosaminyltransferase I, **dadurch gekennzeichnet, dass** das Enzym die in SEQ ID NO: 2 angegebene Aminosäuresequenz umfasst.

16. N-Acetylglucosaminyltransferase I, **dadurch gekennzeichnet, dass** das Enzym die Aminosäuren 74 bis 446 der in SEQ ID NO: 2 angegebenen Aminosäuresequenz umfasst.

17. N-Acetylglucosaminyltransferase I, **dadurch gekennzeichnet, dass** das Enzym die in SEQ ID NO: 4 angegebene Aminosäuresequenz umfasst.

18. Pflanzliche N-Acetylglucosaminyltransferase I, zugänglich aufgrund der Hybridisierung ihres Gens unter stringenten Bedingungen mit einer DNA gemäß einem der Ansprüche 5 bis 10.

19. Von den Enzymen gemäß einem der Ansprüche 14 bis 18 durch Substitution, Deletion, Insertion, und/oder Modifikation einzelner Aminosäuren und/oder kleinerer Gruppen von Aminosäuren und/oder durch N- oder C-terminale Verkürzung und/oder Verlängerung abgeleitete pflanzliche Enzyme oder Proteine, mit der Maßgabe, dass deren Gene unter stringenten Bedingungen mit einer DNA gemäß einem der Ansprüche 5 bis 10 hybridisieren.

20. Mikroorganismus, **dadurch gekennzeichnet, dass** er durch mindestens eine der Nukleotidsequenzen, ausgewählt aus den DNAs, Konstrukten, Vektoren, Plasmiden, Cosmiden, Viren- oder Phagengenomen gemäß einem oder mehreren der Ansprüche 5 bis 13 transformiert ist.

21. Transgene Pflanze, transgener Samen, transgenes Vermehrensrnaterial, Teile transgener Pflanzen oder transformierte Pflanzenzelle, erhältlich durch Integration einer oder mehrerer DNA-Sequenz(en) oder Konstrukt(e) nach einem der Ansprüche 5 bis 11 unter Kontrolle eines in Pflanzen wirksamen Promotors in das Genom einer Pflanze oder durch Infektion durch ein eine oder mehrerer DNA-Sequenz(en) oder Konstrukt(e) nach einem der Ansprüche 5 bis 11 enthaltendes Virus für eine extrachromosomale Propagation und Expression des DNA-Sequenz(en) oder des Konstrukts/der Konstrukte in infiziertem Pflanzengewebe.

22. Transgene Pflanze, transgener Samen, transgenes Vermehrensmaterial, Teile transgener Pflanzen oder transformierte Pflanzenzelle mit fehlender oder verminderter N-Acetylglucosaminyltransferase I-Aktivität, erhältlich durch Integration eines oder mehrerer "antisense"- oder "sense"-Konstrukt (e) nach Anspruch 12 unter Kontrolle eines in Pflanzen wirksamen Promotors in das Genom einer Pflanze oder durch virale Infektion durch ein ein oder mehrere "antisense"- bzw. "sense"-Konstrukt(e) nach Anspruch 12 enthaltendes Virus für eine extrachromosomale Propagation und Transkription von dem oder den "antisense"-Konstrukt(en) oder von dem oder den "sense"-Konstrukten in infiziertem Pflanzengewebe.

## Claims

1. Process for the production of glycoproteins having minimal, homogeneous GlcNAc₂Man₅ residues, which process comprises culturing a transgenic plant, parts of transgenic plants or transformed plant cells and isolating the desired glycoprotein from the cultured material, **characterised in that** the transgenic plant, the parts of transgenic plants or the transformed plant cells has or have been transformed with an antisense construct or a sense construct, comprising an antisense DNA or sense DNA with respect to the DNA sequence of a gene or cDNA for plant N-acetylglucosaminyltransferase I or of a part thereof, in order to eliminate or reduce N-acetylglucosaminyltransferase I activity therein, the antisense or sense construct optionally additionally comprising regulatory sequences for the transcription of the corresponding antisense or sense DNA.

2. Process according to claim 1, **characterised in that** there is used for the transformation an antisense or sense construct with respect to one of the cDNAs coding for N-acetylglucosaminyltransforase I from Solanum tuberosum, Nicotiana tabacum or Arabidopsis thaliana.

3. Process according to claim 2, **characterised in that** for the transformation an antisense or sense construct of one of the DNA sequences shown in SEQ ID No: 1, 3 or 5 is used.

4. Process according to any one of claims 1 to 3, **characterised in that** the transgenic plant used has additionally been transformed with the gene coding for the desired glycoprotein.

5. DNA, **characterised in that** it codes for N-acetylglucosaminyltransferase I from Solanum tuberosum and comprises the nucleotide sequence shown in SEQ ID NO: 1.

6. DNA, **characterised in that** it codes for N-acetylglucosaminyltransferase I from Nicotiana tabacum and comprises the nucleotide sequence shown in SEQ ID NO: 3.

7. DNA coding for N-acetylglucosaminyltransferase I from Arabidopsis thaliana, **characterised in that** the DNA codes for the amino acid sequence shown in SEQ ID NO: 6 or comprises the nucleotide sequence shown in SEQ ID NO: 5.

8. DNA, **characterised in that** it exhibits the complementary nucleotide sequence to the DNA according to claim 5, 6 or 7.

9. DNA, **characterised in that** it can be obtained by substitution, deletion and/or insertion of individual nucleotides or of a plurality of nucleotides and/or by shortening at the 5'- and/or 3'-end of one of the DNAs according to any one of claims 5 to 8, with the proviso that the DNA hybridises under stringent conditions with the starting DNA or its complementary sequence.

10. DNA, **characterised in that** it constitutes a gene or is part of a gene that codes for the plant enzyme N-acetylgtucosaminyltransferase I and hybridises under stringent conditions
- with one of the DNA sequences according to any one of claims 5 to 9 and/or
- with a DNA sequence that has been derived from the amino acid sequence shown in SEQ ID NO: 1, 3 and/or 5, taking into account the degeneracy of the genetic code.

11. , DNA construct, **characterised in that** it comprises one or more of the DNAs according to any one of claims 5 to 10.

12. DNA construct, **characterised in that** it comprises an antisense or sense DNA with respect to the DNA sequence according to any one of claims 5 to 10 and optionally regulatory sequences for the transcription of the antisense or sense DNA.

13. Vector, plasmid, cosmid, viral or phage genome, **characterised in that** it comprises at least one DNA and/or construct according to any one of claims 5 to 12.

14. N-acetylglucosaminyltransferase I from Arabidopsis thaliana, **characterised in that** the enzyme comprises the amino acid sequence shown in SEQ ID NO: 6.

15. N-acetylglucosaminyltransferase I, **characterised in that** the enzyme comprises the amino acid sequence shown in SEQ ID NO: 2.

16. N-acetylglucosaminyltransferase 1, **characterised in that** the enzyme comprises amino acids 74 to 446 of the amino acid sequence shown in SEQ ID NO: 2.

17. N-acetylglucosaminyltransferase I, **characterised in that** the enzyme comprises the amino acid sequence shown in SEQ ID NO: 4.

18. Plant N-acetylglucosaminyltransferase I, obtainable on the basis of the hybridisation of its gene under stringent conditions with a DNA according to any one of claims 5 to 10.

19. Plant enzymes or proteins derived from the enzymes according to any one of claims 14 to 18 by substitution, deletion, insertion and/or modification of individual amino acids and/or of relatively small groups of amino acids and/or by N- or C-terminal shortening and/or lengthening, with the proviso that their genes hybridise under stringent conditions with a DNA according to any one of claims 5 to 10.

20. Microorganism, **characterised in that** it has been transformed by at least one nucleotide sequence selected from the DNAs, constructs, vectors, plasmids, cosmids, viral or phage genomes according to one or more of claims 5 to 13.

21. Transgenic plant, transgenic seed, transgenic propagation material, parts of transgenic plants or transformed plant cell, obtainable by integration of one or more DNA sequence(s) or construct(s) according to any one of claims 5 to 11, under the control of a promoter effective in plants, into the genome of a plant or by infection by a virus containing one or more DNA sequence(s) or construct(s) according to any one of claims 5 to 11, for extrachromosomal propagation and expression of the DNA sequence(s) or construct(s) in infected plant tissue.

22. Transgenic plant, transgenic seed, transgenic propagation material, parts of transgenic plants or transformed plant cell having no or reduced N-acetylglucosaminyltransferase I activity, obtainable by integration of one or more antisense or sense construct(s) according to claim 12, under the control of a promoter effective in plants, into the genome of a plant or by viral infection by a virus containing one or more antisense or sense construct(s) according to claim 12, for extrachromosomal propagation and transcription of the antisense construct(s) or of the sense construct(s) in infected plant tissue.

## Revendications

1. Procédé de production de glycoprotéines comportant des restes minimum homogènes de GlcNAc₂Man₅, d'une manière globale, la culture d'une plante transgénique, de parties de plantes transgéniques ou de cellules de plantes transformées et l'isolement de la glycoprotéine recherchée du matériel de culture, **caractérisé en ce que** la plante transgénique, les parties de plantes transgéniques respectivement les cellules de plantes transformées ayant une construction "antisens" ou une construction "sens", comprenant un ADN "antisens" respectivement un ADN "sens" concernant la séquence d'ADN d'un gène ou d'un cADN de la N-acétylglucosaminyl transférase I ou d'une partie de celles-ci, est respectivement sont transformées pour supprimer ou réduire l'activité N-acétylglucosaminyl transférase I dans celles-ci, la construction "antisens" ou "sens" contenant en plus, le cas échéant, des séquences régulatrices pour la transcription de l'ADN "antisens" ou "sens" correspondant.

2. Procédé selon la revendication 1, **caractérisé en ce que**, pour la transformation d'une construction "antisens" ou "sens" de l'un des cADN, on utilise la N-acétylglucosaminyl transférase I du Solanum tuberosum, Nicotiana tabacum ou Arabidopsis thaliana.

3. Procédé selon la revendication 2, **caractérisé en ce qu'**on utilise, pour la transformation, une construction "antisens" ou "sens" de l'une des séquences ADN indiquées dans SEQ ID NO: 1,3 et 5.

4. Procédé selon l'une quelconque des revendications 1 à 3, **caractérisé en ce que** la plante transgénique mise en oeuvre a été transformée en plus avec le gène codant la glycoprotéine recherchée.

5. ADN, **caractérisé en ce qu'**il code la N-acétylglucosaminyl transférase I à partir du Solanum tuberosum et comporte la séquence de nucléotides indiquée dans SEQ ID NO:1.

6. ADN, **caractérisé en ce qu'**il code la N-acétylglucosaminyl transférase I à partir du Nicotiana tabacum et comporte la séquence de nucléotides indiquée dans SEQ ID NO:3.

7. ADN, qui code la N-acétylglucosaminyl transférase I à partir de l'Arabidopsis thaliana, **caractérisé en ce que** l'ADN code la séquence d'acides aminés indiquée dans SEQ ID NO 6 et comporte la séquence de nucléotides indiquée dans SEQ ID NO:5.

8. ADN, **caractérisé en ce qu'**il présente la séquence de nucléotides complémentaires à l'ADN selon la revendication 5, 6 ou 7.

9. ADN, **caractérisé en ce qu'**il peut être obtenu par substitution, délétion et/ou insertion d'un ou plusieurs nucléotides et/ou raccourcissement sur l'extrémité 5' et/ou 3' de l'un des ADN selon l'une quelconque des revendications 5 à 8, étant précisé que l'ADN est hybridé dans des conditions stringentes avec l'ADN de départ ou avec la séquence complémentaire de celui-ci.

10. ADN, **caractérisé en ce qu'**il représente un gène ou une partie d'un gène qui code l'enzyme **caractérisé en ce qu'**il représente un gène ou une partie d'un gène qui code l'enzyme végétale N-acétylglucosaminyl transférase I et hybridé dans des conditions stringentes
- avec l'une des séquences ADN selon l'une quelconque des revendications 5 à 9 et/ou
- avec une séquence ADN qui a été dérivée des séquences d'acides aminés indiquées dans SEQ ID NO: 1, 3 et/ou 5 compte tenu de la dégénérescence du code génétique.

11. Construction d'ADN, **caractérisée en ce qu'**elle comporte un ou plusieurs des ADN selon l'une quelconque des revendications 5 à 10.

12. Construction d'ADN, **caractérisée en ce qu'**elle comporte un ADN "antisens" ou "sens" par rapport à la séquence d'ADN selon l'une quelconque des revendications 5 à 10 et, le cas échéant, des séquences régulatrices pour la transcription de l'ADN "antisens" respectivement "sens".

13. Vecteur, plasmide, cosmide, génome viral ou du phage, **caractérisé en ce qu'**il comporte au moins un ADN et/ou une construction selon l'une quelconque des revendications 5 à 12.

14. N-acétylglucosaminyl transférase I à partir d'Arabidopsis thaliana, **caractérisée en ce que** l'enzyme comporte la séquence d'acides aminés indiquée dans SEO ID NO:6.

15. N-acétylglucosaminyl transférase I, **caractérisée en ce que** l'enzyme comporte la séquence d'acides aminés indiqués dans SEQ ID NO:2.

16. N-acétylglucosaminyl transférase I, **caractérisée en ce que** l'enzyme comporte les acides aminés 74 à 446 de la séquence d'acides aminés indiquée dans SEQ ID NO:2.

17. N-acétylglucosaminyl transférase I, **caractérisée en ce que** l'enzyme comporte la séquence d'acides aminés indiquée dans SEQ ID NO:4.

18. N-acétytglucosaminyl transférase I végétale, pouvant être obtenue par hybridation de son gène dans des conditions stringentes avec un ADN selon l'une quelconque des revendications 5 à 10.

19. Enzymes ou protéines végétales dérivées des enzymes selon l'une quelconque des revendications 14 à 18 par substitution, délétion, insertion, et/ou modification d'acides aminés individuels et/ou de petits groupes d'acides aminés et/ou par raccourcissement et/ou prolongation sur la terminaison N ou C, étant précisé que leurs gènes s'hybrident, dans des conditions stringentes avec un ADN selon l'une quelconque des revendications 5 à 10.

20. Microorganisme, **caractérisé en ce qu'**il est transformé par au moins une des séquences de nucléotides, choisies dans les ADN, constructions, vecteurs, plasmides, cosmides, génomes viraux ou du phage selon l'une quelconque ou plusieurs des revendications 5 à 13.

21. Plantes transgéniques, graines transgéniques, matériel de multiplication transgénique, parties de plantes transgéniques ou cellule végétale transformée, pouvant être obtenus par intégration, dans le génome d'une plante, d'une ou plusieurs séquence(s) d'ADN ou construction(s) selon l'une quelconque des revendications 5 à 11 sous le contrôle d'un promoteur, actif dans les plantes, ou bien par infection par un virus comportant une ou plusieurs séquence(s) d'ADN ou construction(s) selon l'une quelconque des revendications 5 à 11 pour une propagation et expression extrachromosomiques de la (des) séquence(s) d'ADN ou de la (des) construction(s) dans le tissu infecté de la plante.

22. Plante transgénique, graines transgéniques, matériel de multiplication transgénique, parties de plantes transgéniques ou cellule végétale transformée ayant une activité N-acétylglucosaminyl transférase I manquante ou réduite, pouvant être obtenu(e) par intégration dans le génome d'une plante d'une ou plusieurs construction(s) "antisens" ou "sens" selon la revendication 12 sous le contrôle d'un promoteur, actif dans les plantes, ou bien par infection virale par un virus comportant une ou plusieurs construction(s) "antisens" ou "sens" selon la revendication 12 pour une propagation et transcription extrachromosomiques de la ou des construction(s) "antisens" ou de la ou des construction(s) "sens" dans le tissu végétal infecté.
